(19) **Europäisches Patentamt / European Patent Office / Office européen des brevets**

(11) **EP 4 660 310 A1**

(12) **EUROPEAN PATENT APPLICATION**
published in accordance with Art. 153(4) EPC

(43) Date of publication:
**10.12.2025 Bulletin 2025/50**

(21) Application number: **24830189.7**

(22) Date of filing: **11.05.2024**

(51) International Patent Classification (IPC):
*C12N 15/65* (2006.01)    *C12N 15/66* (2006.01)
*C12N 15/85* (2006.01)    *C12N 5/10* (2006.01)

(52) Cooperative Patent Classification (CPC):
**C12N 5/10; C12N 15/65; C12N 15/66; C12N 15/85;**
**C12N 15/90; G06N 20/00; G16B 20/20**

(86) International application number:
**PCT/CN2024/092536**

(87) International publication number:
**WO 2025/001541 (02.01.2025 Gazette 2025/01)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **30.06.2023 CN 202310802807**

(71) Applicant: **Shenzhen Taili Biotechnology Co., Ltd**
**Shenzhen, Guangdong 518548 (CN)**

(72) Inventors:
• **CHEN, Liang**
**Shenzhen, Guangdong 518548 (CN)**
• **LIN, Longzhi**
**Shenzhen, Guangdong 518548 (CN)**

• **HU, Zhipeng**
**Shenzhen, Guangdong 518548 (CN)**
• **ZHANG, Ning**
**Shenzhen, Guangdong 518548 (CN)**
• **YANG, Xiaoli**
**Shenzhen, Guangdong 518548 (CN)**
• **LI, Wenzheng**
**Shenzhen, Guangdong 518548 (CN)**
• **LIANG, Guolong**
**Shenzhen, Guangdong 518548 (CN)**

(74) Representative: **Valet Patent Services Limited**
**c/o Caya 83713X**
**Am Börstig 5**
**96052 Bamberg (DE)**

Remarks:
The complete document including Reference Table(s) and the Sequence Listing(s) can be downloaded from the EPO website

(54) **METHOD FOR SCREENING SITE-DIRECTED INTEGRATION SITE, VECTOR MICROPARTICLE, SITE-DIRECTED INTEGRATION METHOD AND MICROPARTICLE**

(57) Provided are a method for screening a site-directed integration site, a vector microparticle, a site-directed integration method and a microparticle. Provided are a method for screening a site-directed integration site, a vector microparticle, a site-directed integration method and a microparticle. The objective of the present invention lies in that by integrating a marker gene having recognition sequences upstream and downstream on a vector microparticle, protein expression screening and single clone screening are simultaneously performed so as to obtain a single clone vector having good performance by means of one-step screening, and the marker gene is then replaced when an exogenous gene of interest is expressed so as to greatly ensure the overall stability of the microparticle, which ensure the overall expression performance and stability of the expression microparticle while shortening the development of the expression microparticle, thereby solving the technical problem in the prior art that expression vectors are long in development time or poor in performance. The provided method for screening a site-directed integration site can effectively shorten the development period of expression vectors having high expression and overall stability. The provided vector microparticle has good adaptability, good expression and good passaging stability.

EP 4 660 310 A1

Introducing the selection marker expression RMCE
cassette into a CHO host by random integration

clonal selection
and expansion

Screening for host monoclone with high
selection marker and low copy number

Stability assessment of marker gene cell

Product assessment for stable cells with
high selection marker

The product gene undergoing sequence exchange with the selection marker
expression RMCE cassette in the host through homologous recombination

Proving that the host integration site is a high-expression
and stable site for the product

The candidate host cell strains being subjected to methods
such as sequencing to obtain integration site information

FIG. 5

**Description**

**TECHNICAL FIELD**

The present application belongs to the field of biotechnology, and more specifically, relates to a method for screening a site-directed integration site, a vector microparticle, a site-directed integration method, and a microparticle.

**BACKGROUND ART**

Since the US FDA approved Genetech's recombinant insulin for marketing in 1982, recombinant proteins have been successfully used in scientific research, and diagnosis, treatment and prevention of various diseases. At present, recombinant proteins are mainly produced and expressed by using exogenous protein expression systems. The production of recombinant proteins by using mammalian cells has become the mainstream technology for the expression and production of biotechnology drugs. Currently, most of the biological drugs-including but not limited to polypeptide drugs and protein-based drugs-that have been marketed, are undergoing clinical trials, or are in preclinical research can expressed by mammalian cells, and in the above, Chinese hamster ovary (CHO) cells are the most widely used host cells in the production of recombinant proteins. CHO cells have multiple lines. The CHO cell lines currently used for industrial production mainly include CHO-K1, CHO-DG44, CHO-DXB11 (also known as CHO-DUKX), CHO-S and CHOZN (CHOZN ZFN-Modified CHO Cell Lines), etc., and also CHO-MK which is under development.

A Chinese invention patent (grant publication number CN100381573C) discloses a method for constructing a cell strain containing an amplifiable, highly transcriptionally active site in its genome: using an integration marker vector to insert a marker into an amplifiable, transcriptionally active site in the mammalian genome, and then co-transfecting the mammalian cells with double plasmids, i.e., a targeting expression vector and a recombinase expression vector, to enable the exogenous target gene to be integrated into the marker site of the genome under the action of a site-directed recombination system, rapidly increase the gene copy number through gene amplification, and achieving high expression of the gene. In this patented approach, a recombinase is used to recognize and cleave a pair of recombination signal sequences, such as attP and attB, and the cell strain selected is CHO- dhfr-.

A Chinese invention patent (grant publication number CN114107380A) discloses constructing a cell line by using a bacteriophage Bxb1 site-specific recombinase system, which can greatly reduce cell line development time. First, the site-directed integration site attP is randomly integrated into the CHO-S cell genome by using a random integration method, antibiotic screening is then performed to obtain a CHO-S.attP recombinant cell model, and by using the CHO-S.attp recombinant cell strain as the host cell, DNA carrying the attB site and the target protein gene is site-specifically integrated into the CHO- S.attp cell strain with the aid of Bxb1 recombinase, which is subjected to antibiotic pressure selection to achieve stable and high expression of the target protein.

Currently, when constructing cell lines, an exogenous target gene is inserted into a specific site, and through gene amplification, the gene copy number is rapidly increased to achieve high gene expression. Existing screening methods for known sites include parental cell line construction, parental cell line stability screening, site-directed integration screening of the exogenous target gene, and target cell line stability screening. In the above, the parental cell line construction is one step, while traditional parental cell line construction uses a two-step form of "parental cell line construction + screening" to select the parental cell lines in which the marker gene is inserted into the site. This method involves a relatively long process and requires significant human resources.

Meanwhile, when screening the parental cell line, a marker gene needs to be used, and the marker gene cannot be removed from the genome of the target cell line finally obtained by integration, which affects the overall stability of the target cell line.

**SUMMARY**

In response to the above-mentioned deficiencies or improvement needs in the prior art, the present application provides a method for screening a site-directed integration site, a vector microparticle, a site-directed integration method, and a microparticle, the purposes of which are to integrate a marker gene with upstream and downstream recognition sequences into the vector microparticle and perform monoclonal screening and simultaneously protein expression level screening to obtain a monoclonal vector with good performance through one-step screening, and replace the marker gene when expressing an exogenous target gene, to greatly ensure the overall stability of the microparticle, and shorten the development of an expression microparticle and simultaneously guarantee the overall expression performance and stability of the expression microparticle, thereby solving the technical problem in the prior art of long development time or poor performance of the expression vector.

To achieve the above purposes, according to one aspect of the present application, a method for screening a site-directed integration site is provided, including the following steps:

(1) randomly integrating an exogenous gene containing two recognition sequences and a marker gene between the two recognition sequences, into an endogenous gene of a vector microparticle to obtain a vector microparticle carrying the marker gene;

(2) amplifying and culturing the vector microparticle carrying the marker gene obtained in step (1) to form a to-be-screened vector microparticle pool;

(3) performing monoclonal screening on the to-be-screened vector microparticle pool obtained in step (2), and simultaneously during the monoclonal screening, retaining a monoclonal vector microparticle where the expression level of the corresponding marker gene and/or exogenous target gene is higher than a preset threshold, according to the expression level of the marker gene and/or the exogenous target gene, to obtain a monoclonal vector microparticle with high protein expression level; and

(4) subculturing the monoclonal vector microparticle with high protein expression level obtained in step (3), determining the subculture stability of the monoclonal vector microparticle according to the expression levels of the marker gene in multiple generations of vector microparticles to obtain a monoclonal vector microparticle with high expression level and subculture stability, and determining the insertion position of the exogenous gene on the endogenous gene as the site-directed integration site.

Preferably, in the method for screening a site-directed integration site, the marker gene is of a comparable length to the exogenous target gene to be integrated; and

the marker gene has multiple optional lengths, preferably 0.75k, 1k, 3k, 15k;
preferably, a marker gene of 3k or less is used, and an expression level of the exogenous target gene of other length is predicted through an intelligent learning algorithm.

Preferably, in the method for screening a site-directed integration site, the marker gene is selected from one or more of a neomycin resistance gene, a thymidine kinase gene, a hygromycin phosphotransferase gene, a dihydrofolate reductase gene, a thymidine kinase gene, a glutamine synthetase gene, an asparagine synthetase gene, a tryptophan synthetase gene, a histidinol dehydrogenase gene, an aminoglycoside phosphotransferase gene, a tryptophan synthetase gene, and a fluorescent protein gene; and/or,

the vector microparticle is used to express one or more of an amino acid, a polypeptide, a protein, a nucleotide, a nucleic acid, and a secretion, is one selected from a cell, a bacterium, and a virus, and is commonly a vector cell; the vector cell is an eukaryotic cell; optionally, the eukaryotic cell is a mammalian cell; optionally, the mammalian cell includes a Chinese hamster ovary CHO cell and a human embryonic kidney HEK293 cell; and/or,
the recognition sequences are each independently selected from one or more of the following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a aBxb1 attP sequence, a Bxb1 attB sequence, an attP sequence, an attB sequence, an attP-GA sequence and an attB-GA sequence.

Preferably, in the method for screening a site-directed integration site, in step (3), the monoclonal screening of the vector microparticle is performed using an image processing technology, and the specific solution includes but not limited to deep learning object detection models which are algorithm models of YOLO (You Only Look Once) series and SSD (Single Shot MultiBox Detector) series.

Preferably, in the method for screening a site-directed integration site, the expression level of the marker gene and/or the exogenous target gene in step (3) is predicted by using an intelligent algorithm according to a cell image, specifically including the following steps:

(3-1) imaging the to-be-screened vector microparticle pool obtained in step (2) to acquire to-be-screened vector microparticle images, where preferably the high-throughput scanning imaging is used, and preferably the high-throughput fluorescence scanning imaging is used when the marker gene is a fluorescent protein;

(3-2) performing image processing on the to-be-screened vector microparticle images obtained in step (3-1) to obtain an image of each vector microparticle, and predicting the expression level of the marker gene and/or the exogenous target gene of the vector microparticle by using the intelligent algorithm according to image features, where preferably, the image features used include: cell morphological features and/or optical features, including but not limited to roundness, size, and grayscale of a specific cell site;

preferably, for the expression levels of the marker gene and/or the exogenous target gene of the vector microparticle predicted by multiple intelligent algorithms, normalized discounted cumulative gain (NDCG), an evaluation standard of the ranking algorithm, is used to evaluate the ranking accuracy of the protein expression levels predicted by the multiple intelligent algorithms; according to the rule that the larger the standard normalized discounted cumulative gain, the better the ranking accuracy of the protein expression levels, and the intelligent algorithm with the best ranking accuracy in the predicted protein expression levels is selected;

for a specific intelligent algorithm for predicting the protein expression level, the calculation formula of the standard normalized discounted cumulative gain is as follows:

$$DCG = \sum_{i=1}^{k} \frac{rel(i)}{\log_2(i+1)}$$

$$NDCG = \frac{DCG}{IDCG}$$

where IDCG indicates the DCG value of the actual ranking of protein expression levels of the sample monoclonal vector microparticles, i indicates the predicted ranking number of a monoclonal vector microparticle in the sample monoclonal vector microparticles obtained according to the ranking of the protein expression levels predicted by the intelligent algorithm, *rel(i)* indicates the actual protein expression level of the *i-th* monoclonal vector microparticle; and k indicates the sample size of the sample monoclonal vector microparticles;

where when the predicted protein is a fluorescent protein, *rel(i)* indicates the fluorescence value; and when the predicted protein is a non-fluorescent protein, *rel(i)* indicates the value of the protein expression level tested by means of Western blot, Elisa, mass spectrometry, electrophoresis, etc.; and

(3-3) acquiring, according to the image localization, the vector microparticle where the expression level of the marker gene and/or the exogenous target gene exceeds the preset threshold, and moving to a preset position in a medium for retention.

Preferably, in the method for screening a site-directed integration site, in step (3), a semi-solid medium monoclonal vector microparticle screening is used.

Preferably, in the method for screening a site-directed integration site, in step (4), the subculture stability of the monoclonal vector microparticle is determined by using an intelligent algorithm, including following specific steps:

(4-1) acquiring expression levels of the marker gene of multiple generations of monoclonal vector microparticles;

(4-2) evaluating the differences in the expression levels of the marker gene of the multiple generations of monoclonal vector microparticles by using the intelligent algorithm according to the expression levels of the marker gene of the multiple generations of monoclonal vector microparticles obtained in step (4-1); and

(4-3) determining whether the monoclonal vector microparticle has subculture stability, based on the differences in the expression levels of the marker gene of the multiple generations of monoclonal vector microparticles obtained in step (4-2), according to the principle that the smaller the difference in expression levels, the greater the probability of having subculture stability.

According to another aspect of the present application, there is provided a site-directed integration vector microparticle obtained by screening using the method for screening a site-directed integration site, where an exogenous gene is inserted into an endogenous gene of the vector microparticle; the exogenous gene includes two first recognition sequences and a marker gene expression fragment used for expressing a marker protein and integrated between the two first recognition sequences;

the expression level of the marker gene of the site-directed integration vector microparticle is higher than the preset threshold; and

the site-directed integration vector microparticle has subculture stability.

According to another aspect of the present application, a system for screening a site-directed integration site is provided, where the system is an electronic device and/or a non-transitory computer-readable storage medium;

the electronic device includes a memory, a processor, and a computer program stored on the memory and executable on the processor, where when the processor executes the program, the steps of the method for screening a site-directed integration site provided by the present application are implemented; and

the non-transitory computer-readable storage medium stores a computer program thereon, and when the computer program is executed by the processor, the steps of the method for screening a site-directed integration site provided by the present application are implemented.

According to another aspect of the present application, a site-directed integration method of an exogenous target gene is provided, including the following steps:

acquiring the site-directed integration vector microparticle provided by the present application, performing homologous recombination by using the first recognition sequences in the exogenous gene in the site-directed integration vector microparticle, to recombine at least one exogenous target gene expression fragment into the gene of the site-directed integration vector microparticle and simultaneously remove the marker gene expression fragment.

Preferably, in the site-directed integration method of the exogenous target gene, the at least one exogenous target gene expression fragment is set in a recombinant vector, and the exogenous target gene is site-directed integrated into the site of the site-directed integration vector microparticle through the recombinant vector; the recombinant vector is a lentiviral vector, an adenoviral vector, an adeno-associated viral vector, a herpesvirus vector, a poxvirus vector, a baculovirus vector, a papillomavirus vector, a papovavirus vector, an integrating phage vector, a non-viral vector, a transposon, a transposase, an integrase substrate or a plasmid.

Preferably, in the site-directed integration method of the exogenous target gene, the length of the marker gene expression fragment of the site-directed integration vector microparticle is comparable to that of the at least one exogenous target gene expression fragment.

Preferably, in the site-directed integration method of the exogenous target gene, the performing homologous recombination by using the first recognition sequences of the site-directed integration fragment of the site-directed integration vector microparticle specifically includes:

constructing an exogenous target gene recombinant fragment including the exogenous target gene, where the exogenous gene recombinant fragment contains two second recognition sequences and the at least one exogenous target gene expression fragment; the at least one exogenous target gene expression fragment is between the two second recognition sequences; and the at least one exogenous target gene expression fragment includes a promoter for regulating expression of the exogenous target gene and an exogenous target gene sequence; and

integrating the exogenous gene into the site of the site-directed integration vector microparticle, where the two first recognition sequences on two sides of the marker gene are respectively changed into a third recognition sequence and a fourth recognition sequence; the third recognition sequence is formed by the reaction of the first recognition sequence and the second recognition sequence, and the fourth recognition sequence is formed by the reaction of the first recognition sequence and the second recognition sequence, and the third recognition sequence is different from the fourth recognition sequence.

Preferably, in the site-directed integration method of the exogenous target gene, the third recognition sequence and the fourth recognition sequence both are not changed into the second recognition sequence or the first recognition sequence in the same reaction system.

Preferably, in the site-directed integration method of the exogenous target gene, the third recognition sequence and the fourth recognition sequence are generated by a recombinase, where the recombinase is one of a Bxb1 integrase, a ΦC31 integrase, a Cre recombinase or a FLP recombinase; and/or

the second recognition sequence is independently selected from one or more of the following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a Bxb1 attP sequence, a Bxb1 attB sequence, an attP sequence, an attB sequence, an attP-GA sequence and an attB-GA sequence; and/or,

the third recognition sequence and the recognition sequence are each independently selected from one or more of the following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, an attL sequence and an attR sequence.

Preferably, the site-directed integration method of the exogenous target gene includes the following steps:

S1. acquiring multiple site-directed integration vector microparticles provided by the present application, performing homologous recombination by using the first recognition sequences in the exogenous genes in the site-directed integration vector microparticles, to recombine the exogenous target gene expression fragments into the genes of the site-directed integration vector microparticles and simultaneously remove the marker gene expression fragments to obtain multiple candidate exogenous target gene expression vector microparticles;

S2. amplifying and subculturing the multiple candidate exogenous target gene expression vectors obtained in step S1;

S21. retaining, according to the expression level of the exogenous target gene, monoclonal exogenous target gene expression vector microparticles where the expression level of the corresponding exogenous target gene is higher than a preset threshold;

S22. determining the subculture stabilities of the exogenous target gene expression vector microparticles according to the expression levels of the exogenous target gene of multiple generations of monoclonal exogenous target gene expression vector microparticles; and

obtaining the exogenous target gene expression vector microparticles with high protein expression levels and subculture stabilities.

Preferably, in the site-directed integration method of an exogenous target gene, in step S21, the expression level of the exogenous target gene is predicted by an intelligent learning algorithm; and

the expression level retention of the exogenous target gene is predicted by an intelligent algorithm according to a cell image, specifically including the following steps:

S211. imaging the monoclonal exogenous target gene expression vector microparticles obtained in step S21 to acquire images of candidate monoclonal exogenous target gene expression vector microparticles, where preferably, high-throughput scanning imaging is used; and

S212. predicting the expression levels of the exogenous target gene in the monoclonal exogenous target gene expression vector microparticles by using the intelligent algorithm according to image features, where preferably, the image features used include: cell morphological features and/or optical features, including but not limited to roundness, size, and grayscale of a specific cell site;

preferably, for the expression levels of the exogenous target gene of the monoclonal exogenous target gene expression vector microparticles predicted by multiple intelligent algorithms, normalized discounted cumulative gain (NDCG), an evaluation standard of the ranking algorithm, is used to evaluate the ranking accuracy of the protein expression levels predicted by the multiple intelligent algorithms, according to the rule that the greater the standard normalized discounted cumulative gain, the better the ranking accuracy of the protein expression levels, and the intelligent algorithm with the best ranking accuracy in the predicted protein expression levels is selected;

for a specific intelligent algorithm for predicting the protein expression level, the calculation formula of the standard normalized discounted cumulative gain is as follows:

$$DCG = \sum_{i=1}^{k} \frac{rel(i)}{\log_2(i+1)}$$

$$NDCG = \frac{DCG}{IDCG}$$

where IDCG indicates the DCG value of the actual ranking of the protein expression levels of the sample monoclonal vector microparticles, i indicates the predicted ranking number of a monoclonal vector microparticle in the sample monoclonal vector microparticles obtained according to the ranking of the protein expression levels predicted by the intelligent algorithm, *rel(i)* indicates the actual protein expression level of the *i-th* monoclonal vector microparticle; and k indicates the sample size of the sample monoclonal vector microparticles; and

when the predicted protein is a fluorescent protein, *rel(i)* indicates the fluorescence value; and when the predicted protein is a non-fluorescent protein, *rel(i)* indicates the value of the protein expression level tested by means of Western blot, Elisa, mass spectrometry analysis, electrophoresis, etc.

Preferably, in the site-directed integration method of the exogenous target gene, in step S22, the subculture stabilities of the monoclonal exogenous target gene expression vector microparticles are determined by using an intelligent algorithm, including specific steps as follows:

S221. acquiring the expression levels of the exogenous target gene of multiple generations of the monoclonal exogenous target gene expression vector microparticles;

S222. evaluating the differences in the expression levels of the exogenous target gene in the multiple generations of the monoclonal exogenous target gene expression vector microparticles by using the intelligent algorithm according to the expression levels of the exogenous target gene of the multiple generations of the monoclonal exogenous target gene expression vector microparticles obtained in step S221; and

S223. determining whether the monoclonal exogenous target gene expression vector microparticles have subculture stabilities, based on the differences in the expression levels of the exogenous target gene of the multiple generations of monoclonal exogenous target gene expression vector microparticles obtained in step S222, according to the principle that the smaller the difference in expression levels, the greater the probability of having subculture stability.

According to another aspect of the present application, there is provided an exogenous target gene expression vector microparticle obtained by screening using the site-directed integration method of an exogenous target gene, where the exogenous target gene is inserted into the endogenous gene of the vector microparticle; the exogenous gene includes a third recognition sequence, a fourth recognition sequence, and at least one exogenous target gene expression fragment for expressing a target protein located between the third recognition sequence and the fourth recognition sequence;

the expression level of target gene of the exogenous target gene expression vector microparticle is higher than the preset threshold; and
the exogenous target gene expression vector microparticle has subculture stability.

According to another aspect of the present application, a site-directed integration system of an exogenous target gene is provided, where the system is an electronic device and/or a non-transitory computer-readable storage medium;

the electronic device includes a memory, a processor, and a computer program stored on the memory and executable on the processor, where when the processor executes the program, the steps of the site-directed integration method of an exogenous target gene provided by the present application are implemented; and
the non-transitory computer-readable storage medium stores a computer program thereon, and when the computer program is executed by the processor, the steps of the site-directed integration method of an exogenous target gene provided by the present application are implemented.

In general, the above technical solutions conceived by the present application may achieve the following beneficial effects compared with the prior art.

The present application achieves two effects by integrating an exogenous gene containing two recognition sequences and a marker gene between the two recognition sequences into the endogenous gene of the vector microparticle: on the one hand, monoclonal screening and expression level screening are completed synchronously, achieving that a monoclonal vector with high expression level is obtained in one step; and on the other hand, the marker gene is directly replaced when the exogenous target gene is expressed, without the need to load the marker gene in the final protein expression vector, thereby improving the overall stability of the cell. Combining the above two effects, the method for screening a site-directed integration site of the present application may effectively shorten the development cycle of the expression vector with high expression level and overall stability.

The vector microparticle provided by the present application has good adaptability to different exogenous target genes, and good expression level and subculture stability.

The present application covers the entire process of cell strain selection, from the construction of the plasmid of the target protein to the final determination of the stable, high-yielding and protein-producing cell strain. The entire process is standardized, repeatable and highly operable.

The preferred solution uses artificial intelligence algorithms to evaluate and predict the performance of monoclonal expression vectors, which increases the probability of screening monoclonal expression vectors with good performance in a single culture, and further shortens the development cycle of expression vectors with site-directed integration site overall.

In a preferred solution, the present application utilizes high-throughput scanning of a microscope to obtain cell image data to screen for monoclonal cell strains that highly express the target protein, uses a robotic arm to semi-automatically aspirate and transfer the screened cell strains, and finally, uses an image recognition algorithm to screen out cell strains with stable expression. Compared to other technologies for screening monoclonal cell strains, the present application offers the advantages of higher accuracy and reduced time and effort.

## BRIEF DESCRIPTION OF DRAWINGS

FIG. 1 is a structural schematic diagram of a recombinant plasmid including RMCE, constructed in Example 1 of the present application;
FIG. 2 is a structural schematic diagram of the marker gene expression fragment contained in the recombinant plasmid provided in Example 1 of the present application;
FIG. 3 is a flow chart of the method for screening a site-directed integration site provided in Example 1 of the present application;
FIG. 4 is a diagram showing the detection effect of the object detection algorithm provided in Example 1 of the present application;
FIG. 5 is an operation flow chart provided by Example 2 of the present application;

FIG. 6 is a recombinant plasmid of target genes 1, 2, 4, and 5 provided in Example 2 of the present application;

FIG. 7 is a recombinant plasmid of target gene 3 provided in Example 2 of the present application;

FIG. 8 is a graph showing the comparison result of the expression levels on day 14 of expression vectors of Example 2;

FIG. 9 is a graph showing the results of the 7-day batch culture expression levels of the expression vector in Example 2;

FIG. 10 is an electrophoresis photograph of GBB003-2D8 cell integrated with a target gene 1 in Example 2;

FIG. 11 is an electrophoresis photograph of GBB003-1G2 cell integrated with a target gene 2 in Example 2;

FIG. 12 is an electrophoresis photograph of GBB003-1D6 cell integrated with a target gene 3 in Example 2;

FIG. 13 is a graph showing the test results of the subcultured cell viability stabilities of Y5E6 and Y6F10 as the exogenous target gene expression vector microparticles in Example 2;

FIG. 14 is a graph showing the test results of the cell proliferation rate stabilities of Y5E6 and Y6F10 as the exogenous target gene expression vector microparticles in Example 2;

FIG. 15 is a graph showing the results of the product quality attributes: glycoforms of Y5E6 cell and Y6F10 cell in Example 2;

FIG. 16 is a graph showing the charge variation comparison result of proteins expressed by Y5E6 cell and Y6F10 cell in Example 2;

FIG. 17 is a graph showing the results of sodium dodecyl sulfate-polyacrylamide gel electrophoresis detection of Y5E6 cell and Y6F10 cell in Example 2;

FIG. 18 is a graph showing the concentration results of antibodies expressed by the target gene 5 in GBB003 cell integrated with the target gene 5 in Example 2;

FIG. 19 is a graph showing the results of sodium dodecyl sulfate-polyacrylamide gel electrophoresis detection of GBB003 cell integrated with the target gene 5 in Example 2;

FIG. 20 is a graph showing the test results of 90-day subculture growth stability of GBB-01 cell provided in Example 3;

FIG. 21 is a structural schematic diagram of the exogenous target gene 6 expression fragment in Example 3 into which the target gene 6 fragment is cloned;

FIG. 22 is a structural schematic diagram of the Bxb1 integrase plasmid provided in Example 3;

FIG. 23 is a graph showing the expression level result of the integrated exogenous target gene 6 expression vector microparticle GBB-01-H8 cell provided in Example 3;

FIG. 24 is a graph showing the batch expression level results of GBB-01-H8 cell provided in Example 3;

FIG. 25 is a graph showing the batch specific productivity results of GBB-01-H8 cell provided in Example 3;

FIG. 26 is a graph showing the glycosylation profile comparison result of GBB-01-H8 cell in Example 3;

FIG. 27 is a graph showing the charge variation comparison result of GBB-01-H8 cell provided in Example 3;

FIG. 28 is an electrophoresis result diagram of the GBB-01-H8 cell genome provided in Example 3;

FIG. 29 is a graph showing the expression level comparison result of the integrated exogenous target gene 3 GBBc001 cell provided in Example 4;

FIG. 30 is a graph showing the expression level comparison result of the integrated exogenous target gene 1 GBBc001 cell provided in Example 4;

FIG. 31 is a graph showing the expression level comparison result of the integrated exogenous target gene 3 GBBc001-14 cell provided in Example 4;

FIG. 32 is a graph showing the result of sodium dodecyl sulfate-polyacrylamide gel electrophoresis detection of GBBc001-14 cell provided in Example 4;

FIG. 33 is a graph showing the result of sodium dodecyl sulfate- polyacrylamide gel electrophoresis detection of GBBc001-3 cell provided in Example 4; and

FIG. 34 is a structural schematic diagram of the system for screening a site-directed integration site provided in Example 5.

## DETAILED DESCRIPTION OF EMBODIMENTS

In order to make the purposes, technical solutions and advantages of the present application more clearly and understandable, the present application will be further described in detail below with reference to the following embodiments. It should be understood that the specific embodiments described herein are merely for the purpose of explaining the present application and are not intended to limit the present application. In addition, the technical features involved in the embodiments of the present application described below may be combined with each other as long as they do not conflict with each other.

The preset threshold herein refers to a fixed value, or a ranking range value that is greater than a certain ranking range value in an existing numerical range in which the values are sorted from largest to smallest, or a ranking value that is greater than a certain ranking value in an existing numerical range in which the values are sorted from largest to smallest.

The recognition sequence herein includes but not limited to homology arm sequence, other sequence that can be recognized, or other homologous recombination sequence.

Herein, "simultaneously during the monoclonal screening, retaining a monoclonal vector microparticle where the expression level of the corresponding marker gene and/or exogenous target gene is higher than a preset threshold, according to the expression level of the marker gene and/or the exogenous target gene" refers to either "simultaneously during the monoclonal screening, retaining a monoclonal vector microparticle where the expression level of the marker gene is higher than a preset threshold, according to the expression level of the marker gene " or "simultaneously during the monoclonal screening, retaining a monoclonal vector microparticle where the expression level of the exogenous target gene is higher than a preset threshold, according to the expression level of the exogenous target gene".

The method for screening a site-directed integration site provided by the present application includes the following steps:

(1) randomly integrating an exogenous gene containing two recognition sequences and a marker gene between the two recognition sequences into an endogenous gene of a vector microparticle to obtain a vector microparticle carrying the marker gene,

where the recognition sequence includes but not limited to the homology arm sequence, other sequence that can be recognized or other homologous recombination sequence. In this embodiment, the recognition sequence refers to the homology arm sequence.

Because the expression level of protein and the stability of the vector microparticle integrated with an exogenous target gene are strongly correlated with the size of the exogenous target gene and the integration site thereof, in order to ensure that the exogenous target gene expression level and stability of the constructed vector microparticle integrated with the exogenous target gene meet screening expectations, the marker gene is of a length comparable to that of the exogenous target gene to be integrated; and in the absence of a clear exogenous target gene, multiple marker genes of representative lengths may be selected respectively for screening, where the typical marker gene lengths are 0.75k, 1k, 3k, and 15k; and in a preferred solution, a marker gene of a small length (3k or less), for example, a marker gene of 0.75k, may be used, and the expression levels of exogenous target genes of other lengths are predicted through an intelligent learning algorithm, significantly reducing costs.

The marker gene is selected from one or more of a neomycin resistance gene, a thymidine kinase gene, a hygromycin phosphotransferase gene, a dihydrofolate reductase gene, a thymidine kinase gene, a glutamine synthetase gene, an asparagine synthetase gene, a tryptophan synthetase gene, a histidinol dehydrogenase gene, an aminoglycoside phosphotransferase gene, a tryptophan synthetase gene, and a fluorescent protein gene.

The vector microparticle is used to express one or more of an amino acid, a polypeptide, a protein, a nucleotide, a nucleic acid, and a secretion, is one selected from a cell, a bacterium, and a virus, and is commonly a vector cell; the vector cell is an eukaryotic cells; optionally, the eukaryotic cell is a mammalian cell; optionally, the mammalian cell includes a Chinese hamster ovary CHO cell and a human embryonic kidney HEK293 cell.

(2) amplifying and culturing the vector microparticle carrying the marker gene obtained in step (1) to form a to-be-screened vector microparticle pool; and

(3) performing monoclonal screening on the to-be-screened vector microparticle pool obtained in step (2), and simultaneously during the monoclonal screening, retaining a monoclonal vector microparticle where the expression level of the corresponding marker gene and/or exogenous target gene is higher than the preset threshold, according to the expression level of the marker gene and/or exogenous target gene to obtain the monoclonal vector microparticle with high protein expression level, where in a preferred solution, the monoclonal screening of the vector microparticle is performed using an image processing technology, and specific solution includes but not limited to deep learning object detection models which are algorithm models of YOLO (You Only Look Once) series and SSD (Single Shot MultiBox Detector) series.

In a preferred solution, the expression level of the marker gene and/or the exogenous target gene is predicted by using an intelligent algorithm according to the cell image, specifically including the following steps:

(3-1) imaging the to-be-screened vector microparticle pool obtained in step (2) to acquire to-be-screened vector microparticle images, where preferably, the high-throughput scanning imaging is used, and preferably, the high-throughput fluorescence scanning imaging is used when the marker gene is a fluorescent protein;

(3-2) performing image processing on the to-be-screened vector microparticle images obtained in step (3-1) to obtain an image of each vector microparticle, and predicting the expression level of the marker gene and/or the exogenous target gene of the vector microparticle by using the intelligent algorithm according to the image features, where preferably, the image features used include: cell morphological features and/or optical features, including but not limited to roundness, size, and grayscale of a specific cell site;

preferably, for the expression levels of the marker gene and/or the exogenous target gene of the vector microparticle predicted by the multiple intelligent algorithms, normalized discounted cumulative gain (NDCG), an evaluation standard of the ranking algorithm, is used to evaluate the ranking accuracy of the protein expression levels predicted by the multiple intelligent algorithms; according to the rule that the larger the standard normalized discounted cumulative gain, the better the ranking accuracy of the protein expression levels, and the intelligent algorithm with the best ranking accuracy in the predicted protein expression levels is selected;

for a specific intelligent algorithm for predicting the protein expression level, the calculation formula of the standard normalized discounted cumulative gain is as follows:

$$DCG = \sum_{i=1}^{k} \frac{rel(i)}{\log_2(i+1)}$$

$$NDCG = \frac{DCG}{IDCG}$$

where IDCG indicates the DCG value of the actual ranking of the protein expression levels of the sample monoclonal vector microparticles, i indicates the predicted ranking number of a monoclonal vector microparticle in the sample monoclonal vector microparticles obtained according to the ranking of the protein expression levels predicted by the intelligent algorithm, *rel(i)* indicates the actual protein expression level of the *i-th* monoclonal vector microparticle; and k indicates the sample size of the sample monoclonal vector microparticles;

where when the predicted protein is a fluorescent protein, *rel(i)* indicates the fluorescence value; and when the predicted protein is a non-fluorescent protein, *rel(i)* indicates the value of the protein expression level tested by means of Western blot, Elisa, mass spectrometry, electrophoresis, etc.;

(3-3) acquiring, according to the image localization, the vector microparticle in which the expression level of the marker gene and/or the exogenous target gene exceeds the preset threshold, and moving to a preset position in a medium for retention,

where since there are differences in the sequence and other properties of the marker gene and the exogenous target gene, the intelligent algorithm is used to learn and train the differences between the two, and then the expression level of the exogenous target gene is predicted, which may improve the adaptability of the expression of the exogenous target gene at the selected integration site to a certain extent, thereby shortening the overall development cycle and reducing development costs.

Limiting dilution method, flow cytometry cell sorting, and semi-solid medium screening are generally used for the screening of the monoclonal cell vector microparticle. The limiting dilution method requires extensive experiments, and the flow cytometry cell sorting uses pressure operation, which has a low vector microparticle survival rate and a high risk of contamination. The present application preferably uses a semi-solid medium monoclonal vector microparticle screening, enabling mechanical automation.

The preset threshold for the expression level of the marker gene and/or exogenous target gene may be determined according to the experimental requirements for protein expression level. For example, if the goal is to require the protein expression level to exceed a certain determined value, the determined value may be used as the preset threshold; alternatively, it may be determined according to the relative protein expression level, for example, a top fixed proportion or top fixed ranking of the expression level of the marker gene and/or exogenous target gene in descending order of expression levels.

(4) subculturing the monoclonal vector microparticle with high protein expression level obtained in step (3), and determining the subculture stability of the monoclonal vector microparticle according to the expression levels of the marker gene in multiple generations of vector microparticles to obtain the monoclonal vector microparticle with high expression level and subculture stability, and determining the insertion position of the exogenous gene on the endogenous gene as the site-directed integration site.

In the preferred solution, the subculture stability of the monoclonal vector microparticle is determined by using an intelligent algorithm, including following specific steps:

(4-1) acquiring the expression levels of the marker gene of multiple generations of monoclonal vector microparticles;

(4-2) evaluating the differences in the expression levels of the marker gene of the multiple generations of monoclonal vector microparticles by using the intelligent algorithm according to the expression levels of the marker gene of the multiple generations of monoclonal vector microparticles obtained in step (4-1); and

(4-3) determining whether the monoclonal vector microparticle has subculture stability, based on the differences in the expression levels of the marker gene of the multiple generations of monoclonal vector microparticles obtained in step (4-2), according to the principle that the smaller the difference in expression levels, the greater the probability of having subculture stability.

For the site-directed integration vector microparticle provided by the present application, an exogenous gene is inserted into the endogenous gene of the vector microparticle; and the exogenous gene includes two first recognition sequences and a marker gene expression fragment used for expressing a marker protein and integrated between the two first recognition sequences;

the expression level of the marker gene of the site-directed integration vector microparticle is higher than the preset threshold; and
the site-directed integration vector microparticle has subculture stability.

In this embodiment, the first recognition sequence refers to the first homology arm sequence.
The marker gene is selected from one or more of a neomycin resistance gene, a thymidine kinase gene, a hygromycin phosphotransferase gene, a dihydrofolate reductase gene, a thymidine kinase gene, a glutamine synthetase gene, an asparagine synthetase gene, a tryptophan synthetase gene, a histidinol dehydrogenase gene, an aminoglycoside phosphotransferase gene, a tryptophan synthetase gene, and a fluorescent protein gene.
The exogenous gene containing two recognition sequences and a marker gene between the two recognition sequences contains a marker gene expression fragment, where the marker gene expression fragment has a promoter. The promoter is a CMV promoter, a SV40 promoter, an RSV promoter, a $\beta$-globin promoter, a UBC promoter, an EF1a promoter, a ubiquitin promoter, a $\beta$-actin promoter, a PGK1 promoter, a Rosa26 promoter, a HSP70 promoter, a GAPDH promoter, an Eif4A1 promoter, an Egr1 promoter, a FerH promoter, an SM22$\alpha$ promoter, or an Endothelin-1 promoter.
The first recognition sequence is independently selected from one or more of the following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a Bxb1 attP sequence, a Bxb1 attB sequence, an attP sequence, an attB sequence, an attP-GA sequence and an attB-GA sequence.
For a system for screening a site-directed integration site provided by the present application, the system is an electronic device and/or a non-transient computer-readable storage medium;

the electronic device includes a memory, a processor, and a computer program stored on the memory and executable on the processor, where when the processor executes the program, the steps of the method for screening a site-directed integration site are implemented; and
the non-transitory computer-readable storage medium stores a computer program thereon, and when the computer program is executed by the processor, the steps of the method for screening site-directed integration sites are implemented.

The site-directed integration method of an exogenous target gene provided by the present application includes the following steps:
acquiring the site-directed integration vector microparticle provided by the present application, performing homologous recombination by using the first recognition sequences in the exogenous gene in the site-directed integration vector microparticle, to recombine the expression fragment into the gene of the site-directed integration vector microparticle and simultaneously remove the exogenous target gene expression fragment of the marker gene.
In a preferred solution, the specific method for screening all site-directed integration vectors for the exogenous target gene is as follows:

S1. acquiring multiple site-directed integration vector microparticles provided by the present application, performing homologous recombination by using the first recognition sequences in the exogenous genes in the site-directed integration vector microparticles, to recombine the exogenous target gene expression fragments into the genes of the site-directed integration vector microparticles and simultaneously remove the marker gene expression fragments to obtain multiple candidate exogenous target gene expression vector microparticles;
S2. amplifying and subculturing the multiple candidate exogenous target gene expression vectors obtained in step S1;
S21. retaining, according to the expression level of the exogenous target gene, monoclonal exogenous target gene expression vector microparticles where the expression level of the corresponding exogenous target gene is higher than a preset threshold, where preferably, the expression level retention of the exogenous target gene is predicted by an intelligent algorithm according to a cell image, specifically including the following steps:

S211. imaging the monoclonal exogenous target gene expression vector microparticles obtained in step S21 to acquire images of the candidate monoclonal exogenous target gene expression vector microparticles, where preferably, high-throughput scanning imaging is used; and

S212. predicting the expression levels of the exogenous target gene in the monoclonal exogenous target gene expression vector microparticles by using the intelligent algorithm according to the image features, where preferably, the image features used include: cell morphological features and/or optical features, including but not limited to roundness, size, and grayscale of a specific cell site;

preferably, for the expression levels of the exogenous target gene of the monoclonal exogenous target gene expression vector microparticles predicted by multiple intelligent algorithms, normalized discounted cumulative gain (NDCG), an evaluation standard of the ranking algorithm, is used to evaluate the ranking accuracy of the protein expression levels predicted by the multiple intelligent algorithms, according to the rule that the greater the standard normalized discounted cumulative gain, the better the ranking accuracy of the protein expression levels, and the intelligent algorithm with the best ranking accuracy in the predicted protein expression levels is selected; for a specific intelligent algorithm for predicting the protein expression level, the calculation formula of the standard normalized discounted cumulative gain is as follows:

$$DCG = \sum_{i=1}^{k} \frac{rel(i)}{\log_2(i+1)}$$

$$NDCG = \frac{DCG}{IDCG}$$

where IDCG indicates the DCG value of the actual ranking of the protein expression levels of the sample monoclonal vector microparticles, i indicates the predicted ranking number of a monoclonal vector microparticle in the sample monoclonal vector microparticles obtained according to the ranking of protein expression levels predicted by the intelligent algorithm, *rel(i)* indicates the actual protein expression level of the *i-th* monoclonal vector microparticle; and k indicates the sample size of the sample monoclonal vector microparticles; and when the predicted protein is a fluorescent protein, *rel(i)* indicates the fluorescence value; and when the predicted protein is a non-fluorescent protein, *rel(i)* indicates the value of the protein expression level tested by means of Western blot, Elisa, mass spectrometry, electrophoresis, etc.

S22. determining the subculture stabilities of the exogenous target gene expression vectors according to the expression levels of the exogenous target gene of multiple generations of monoclonal exogenous target gene expression vector microparticles;

where the subculture stabilities of the monoclonal exogenous target gene expression vector microparticles are determined by using an intelligent algorithm, including the specific steps:

S221. acquiring the expression levels of the exogenous target gene of multiple generation of the monoclonal exogenous target gene expression vector microparticles;

S222. evaluating the differences in the expression levels of the exogenous target gene of the multiple generations of the monoclonal exogenous target gene expression vector microparticles by using the intelligent algorithm according to the expression levels of the exogenous target gene of the multiple generations of the monoclonal exogenous target gene expression vector microparticles obtained in step S221; and

S223. determining whether the monoclonal exogenous target gene expression vector microparticles have subculture stabilities, based on the differences in the expression levels of the exogenous target gene of the multiple generations of monoclonal exogenous target gene expression vector microparticles obtained in step S222, according to the principle that the smaller the difference in expression levels, the greater the probability of having subculture stability.

The exogenous target gene expression vector microparticles which have high protein expression levels and subculture stabilities are obtained.

The exogenous target gene expression fragment is set in a recombinant vector, and the exogenous target gene is site-specifically integrated into the site of the site-directed integration vector microparticle through the recombinant vector; the recombinant vector is a lentiviral vector, an adenoviral vector, an adeno-associated viral vector, a herpesvirus vector, a poxvirus vector, a baculovirus vector, a papillomavirus vector, a papovavirus vector, an integrating phage vector, a non-viral vector, a transposon, a transposase, an integrase substrate or a plasmid.

The length of the marker gene expression fragment of the site-directed integration vector microparticle is comparable to that of the exogenous target gene expression fragment.

The performing homologous recombination by using the first recognition sequences of the site-directed integration fragment of the site-directed integration vector microparticle specifically includes:

constructing an exogenous target gene recombinant fragment including the exogenous target gene, where the exogenous gene recombinant fragment contains two second recognition sequences and at least one exogenous target gene expression fragment, the at least one exogenous target gene expression fragment is between the two second recognition sequences; and the at least one exogenous target gene expression fragment includes a promoter for regulating expression of the exogenous target gene, and an exogenous target gene sequence; and
integrating the exogenous gene into the site of the site-directed integration vector microparticle, where the two first recognition sequences on two sides of the marker gene are respectively changed into a third recognition sequence and a fourth recognition sequence; the third recognition sequence is formed by the reaction of the first recognition sequence and the second recognition sequence, and the fourth recognition sequence is formed by the reaction of the first recognition sequence and the second recognition sequence, and the third recognition sequence is different from the fourth recognition sequence.

In a preferred solution, the third recognition sequence and the fourth recognition sequence both are not changed into the second recognition sequence or the first recognition sequence in the same reaction system.
The third recognition sequence and the fourth recognition sequence are generated by a recombinase, where the recombinase is one of a Bxb1 integrase, a ΦC31 integrase, a Cre recombinase or a FLP recombinase; and/or

the second recognition sequence is independently selected from one or more of the following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a Bxb1 attP sequence, a Bxb1 attB sequence, an attP sequence, an attB sequence, an attP-GA sequence and an attB-GA sequence; and/or,
the third recognition sequence and the recognition sequence are independently selected from one or more of the following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, an attL and an attR sequence.

In this embodiment, the first recognition sequence refers to the first homology arm sequence, the second recognition sequence refers to the second homology arm sequence, the third recognition sequence refers to the third homology arm sequence, and the fourth recognition sequence refers to the fourth homology arm sequence; the third recognition sequence is formed by the reaction of the first homology arm located on the left side of the marker gene in the marker gene expression fragment and the second homology arm located on the left side of the exogenous target gene in the exogenous target gene expression fragment, and the fourth recognition sequence is formed by the reaction of the first homology arm located on the right side of the marker gene in the marker gene expression fragment and the second homology arm located on the right side of the exogenous target gene in the exogenous target gene expression fragment.
For the exogenous target gene expression vector microparticle obtained by screening using the site-directed integration method of an exogenous target gene provided by the present application, the exogenous target gene is inserted into the endogenous gene of the vector microparticle; the exogenous gene includes a third recognition sequence, a fourth recognition sequence, and at least one exogenous target gene expression fragment used for expressing the target protein and arranged between the third recognition sequence and the fourth recognition sequence;

the expression level of the target gene of the exogenous target gene expression vector microparticle is higher than the preset threshold; and
the exogenous target gene expression vector microparticle has subculture stability.

The exogenous target gene expression fragment has a promoter, and the promoter is a CMV promoter, a SV40 promoter, an RSV promoter, a β-globin promoter, a UBC promoter, an EF1a promoter, a ubiquitin promoter, a β-actin promoter, a PGK1 promote, a Rosa26 promote, a HSP70 promoter, a GAPDH promote, an Eif4A1 promoter, an Egr1 promoter, a FerH promoter, a SM22α promoter or an Endothelin-1 promoter.
The third recognition sequence and the fourth recognition sequence are generated by a recombinase, and the recombinase is one of a Bxb1 integrase, a ΦC31 integrase, a Cre recombinase or a FLP recombinase.
The third recognition sequence and the recognition sequence are independently selected from one or more of the following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, an attL and an attR sequence.

In this embodiment, the first recognition sequence refers to the attP sequence, the second recognition sequence refers to the attB sequence, the third recognition sequence refers to the attL sequence, and the fourth recognition sequence refers to the attR sequence.

For a site-directed integration system of an exogenous target gene provided by the present application, the system is an electronic device and/or a non-transient computer-readable storage medium;

the electronic device includes a memory, a processor, and a computer program stored on the memory and executable on the processor, where when the processor executes the program, the steps of the site-directed integration method of an exogenous target gene as described above are implemented; and

the non-transitory computer-readable storage medium stores a computer program thereon, and when the computer program is executed by a processor, the steps of the site-directed integration method of an exogenous target gene as described above are implemented.

The following are examples.

Example 1: Method for screening a site-directed integration site, preparation of site-directed integration vector microparticle, site-directed integration method of exogenous target genes, and preparation of exogenous target gene expression vector microparticle

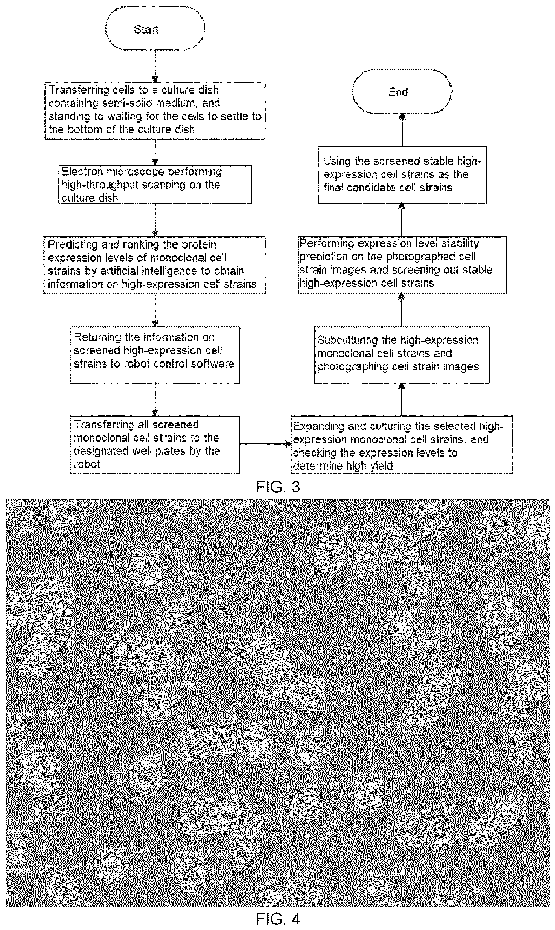

The method for screening a site-directed integration site provided in Example 1, as shown in FIG. 3, included the following steps:

(1) randomly integrating a marker gene with homology arms at two ends into the endogenous gene of the vector microparticle to obtain a vector microparticle carrying the marker gene.

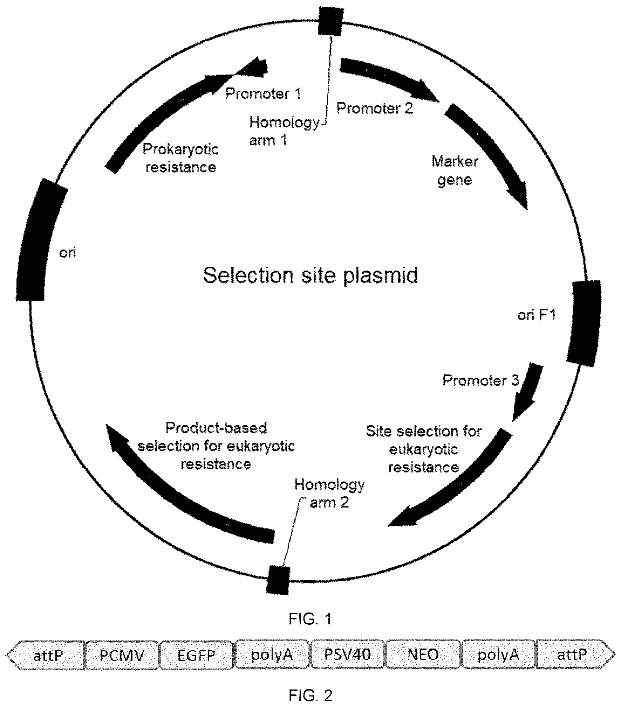

In Example 1, the green fluorescent protein gene (EGFP, Enhanced Green Fluorescent Protein) was used as a screening marker gene, and the attP sequence was used as a homology arm to construct a recombinant plasmid containing RMCE (recombinase-mediated cassette exchange), as shown in FIG. 1 below. The marker gene expression fragment contained in the recombinant plasmid used for performing random integration is as shown in FIG. 2.

After linearizing the constructed plasmid, the linear DNA was purified and recovered; $3 \times 10^6$ CHO-K1 cells were transferred to a 1.5mL centrifuge tube and centrifuged at 1000 rpm for 5 min at a room temperature, and the supernatant was discarded; the cells were resuspended in 100 $\mu$L of R Buffer, which was a resuspension buffer dedicated for a cell electroporation instrument, under electroporation conditions: 1575V, 10ms, 3 cycles, $3 \times 10^6$ cells; and 15 $\mu$g of plasmid. The fluorescent protein gene with attP sequences at two ends is randomly integrated into the endogenous gene on the vector microparticle. The constructed plasmid integrated into the endogenous gene was the exogenous gene, which integrated the marker gene expression fragment as shown in FIG. 2 into the endogenous gene. The vector microparticle was a cell.

The pressurizing reagent was G418, the pressurizing concentration was 800 $\mu$g/ml after two days, the 96-well plate was plated at a density of 10,000 cells per well, with at least 200 $\mu$l of medium per well; the plate was checked after ten days, enrichment was performed when a large number of fluorescent cell clusters were observed, the enrichment methods included, but not limited to manual enrichment. During enrichment, the principle of "one well to one well" should be followed, and cells from multiple wells must not be enriched into a single well.

(2) amplifying and culturing the vector microparticle carrying the marker gene obtained in step (1) to form a to-be-screen vector microparticle pool; where specific steps included:

observing the growth of enriched cells at all times, and expanding the cells after the confluency reaches 50% or above, where there were two expansion methods: 96-well plate-24-well plate-6-well plate and 96-well plate-6-well plate; expanding to a shake flask stage and then subculturing for 2-3 generations to form a stable cell pool, which was used for monoclonal screening.

(3) performing monoclonal screening on the to-be-screen vector microparticle pool obtained in step (2), and simultaneously during the monoclonal screening, retaining a monoclonal vector microparticle where the expression level of the marker gene and/or the exogenous target gene was higher than a preset threshold, according to the expression level of the marker gene and/or the exogenous target gene to obtain the monoclonal vector microparticle with high protein expression level;

where the formation of monoclonal cells and the screening of stable fluorescence were achieved through artificial intelligence, as shown in FIG. 3 below:

the specific steps were described as a-h:

(3-1) imaging the to-be-screened vector microparticle pool obtained in step (2) to acquire to-be-screened vector microparticle images, where preferably, high-throughput scanning imaging was used, and when the marker gene was a fluorescent protein, high-throughput fluorescence scanning imaging was preferably used;

a) diluting the cells in the stable cell pool to a certain concentration and then inoculating into a culture dish containing semi-solid medium, where the cells was ensured to be approximately evenly distributed in all positions of the small dish during the inoculation process; and standing for about half an hour, to wait for the cells to settle to the bottom of the culture dish;

b) transferring the culture dish onto the microscope stage, and performing high-throughput scanning on the cells in the culture dish by the microscope;

(3-2) performing image processing on the to-be-screened vector microparticle images obtained in step (3-1) to obtain an image of each vector microparticle, and predicting the expression level of the marker gene and/or the exogenous target gene of the vector microparticle based on the image features; and

c) uploading the microscope-scanned image to a server for artificial intelligence image analysis, where the analysis process included monoclonal cell strain detection, protein expression level prediction of monoclonal cell strains, protein expression level ranking, and Nos. and localization of the screened high-protein-expressing cell strains, the protein expression level prediction of the monoclonal cell strain may be either fluorescence-based or non-fluorescence-based and the following step c described the non-fluorescence-based protein expression level prediction.

In step c, the monoclonal cell strain object detection was performed based on image processing technology. In this example, the YOLOv8 object detection algorithm was used, and the actual detection effect reached mAP 94.2%, as shown in FIG. 4.

The object detection model for monoclonal cell strains in step c was consistent with commonly used deep learning object detection model, so it was not described in detail. Specifically, the cell images must be annotated first. To improve the prediction accuracy of the algorithm, the bounding boxes of all monoclonal cell strains and adherent cell strains were annotated as the object bounding boxes (ground truth) for model output loss calculation. After algorithm training, the model learned the ability of extracting bounding box information of monoclonal cell strains. In practical application scenarios, in step c, the trained model may predict the bounding boxes of monoclonal cell strains in images.

Because the exogenous gene of different length may be inserted into the screen site and the site was used to express the exogenous gene, and the length of the exogenous gene inserted within the site was strongly correlated with protein expression level, several groups of fluorescent protein marker genes of different deoxyribonucleic acid lengths were selected. In this example, four exogenous gene expression fragments of approximate lengths of 0.75k, 1k, 3k, and 15k were used. These exogenous genes included genes capable of expressing fluorescent proteins and/or genes capable of expressing other proteins. By testing the expression of fluorescent proteins and other proteins, images of cells expressing the exogenous genes of similar lengths, 0.75k, 1k, 3k, or 15k, were selected for artificial intelligence image learning. The case where the length of the exogenous gene was 0.75k corresponds to a case where the exogenous gene at a specific site in the target cell strain may express fluorescent protein and the length of the inserted exogenous gene expression fragment was approximately 0.75k. The case where the length of the inserted exogenous gene was 1K corresponds to a case where the target gene at a specific site in the target cell strain was a double-stranded DNA and the length of the exogenous gene expression fragment was approximately 1k. The case where the length of the inserted exogenous gene was 3K corresponds to a case where the target gene at a specific site in the target cell strain was a monoclonal antibody and the length of the exogenous gene expression fragment ranging from approximately 3k to 7k. The case where the length of the inserted gene was 15K corresponds to a case where the target gene at a specific site in the target cell strain was used for expressing a multivalent antibody or a multi-fragment inserted structure and the length of the exogenous gene expression fragment was approximately 15k.

Step c provided four groups of expression level prediction models of different lengths, each expression level prediction model corresponding to a set of fluorescence value labels. Artificial intelligence image learning was performed using historical images of CHO cells with inserted exogenous target genes of four different lengths, to generate four prediction models respectively for high expression prediction analysis. During the process of site application, the exogenous target gene expression fragments were replaced with exogenous target gene expression fragments of similar lengths. During the construction of CHO cells as historical images, exogenous genes of different lengths may be used as marker gene expression fragments to replace the exogenous genes at high expression sites. The prediction model was then used to determine whether the site was still a high expression site under the protein expression conditions of the inserted genes of different lengths. If it was predicted to be the high expression site after the gene was replaced by each inserted gene of different length as marker gene, the site may be determined to be a protein expression site suitable for exogenous target gene expression fragments of different lengths. In this process, the marker gene may use other proteins that may produce fluorescence, or may use other proteins, with the fluorescence value after expression or the detected protein expression level of the actual marker gene as the label. In step c, historical images of CHO cells in which the length of the deoxyribonucleotides of the inserted exogenous gene was the same with or similar to the length of the DNA sequence of the inserted exogenous gene of the green fluorescent protein gene (EGFP) were used to perform high expression prediction analysis by using a prediction model generated by artificial intelligence image learning.

For the fluorescent protein expression level prediction based on the monoclonal cell image in step c, the SqueezeNet deep learning network and the MSE Loss loss function were used in this example, and the expression level of the exogenous gene or marker gene in the monoclonal cell image was used as the label of the regression algorithm.

For the expression levels of the exogenous target gene of the monoclonal exogenous target gene expression vector microparticle predicted by multiple intelligent algorithms, normalized discounted cumulative gain (NDCG), an evaluation standard of the ranking algorithm, was used to evaluate the ranking accuracy of the protein expression levels predicted by the multiple intelligent algorithms, according to the rule that the greater the standard normalized discounted cumulative gain, the better the ranking accuracy of the protein expression levels, and the intelligent algorithm with the best ranking accuracy in the predicted protein expression levels was selected;

for a specific intelligent algorithm for predicting the protein expression level, the calculation formula of the standard normalized discounted cumulative gain was as follows:

$$DCG = \sum_{i=1}^{k} \frac{rel(i)}{\log_2(i+1)}$$

$$NDCG = \frac{DCG}{IDCG}$$

where IDCG indicated the DCG value of the actual ranking of the protein expression levels of the sample monoclonal vector microparticles, $i$ indicated the predicted ranking number of a monoclonal vector microparticle in the sample monoclonal vector microparticles obtained according to the ranking of protein expression levels predicted by the intelligent algorithm, $rel(i)$ indicated the actual protein expression level of the $i$-th monoclonal vector microparticle; and k indicated the sample size of the sample monoclonal vector microparticles; and

when the predicted protein was a fluorescent protein, $rel(i)$ indicated the fluorescence value; and when the predicted protein was a non-fluorescent protein, $rel(i)$ indicated the value of the protein expression level tested by means of Western blot, Elisa, mass spectrometry, electrophoresis, etc.

Specifically, in this example, cell images were used to predict the expression levels, according to the ranking of the predicted expression levels and compared with the ranking result of the actual fluorescence values, NDCG = 0.89. Reference for the expression level prediction model in the present application was made to the Chinese patent document, "CELL SCREENING METHOD AND APPARATUS BASED ON CONVOLUTIONAL NEURAL NETWORK".

(3-3) acquiring, according to the image localization, the vector microparticle where the expression level of the marker gene and/or the exogenous target gene exceeded a preset threshold, and moving to a preset position in the medium for retention, where the preset threshold referred to the top fixed proportion of the expression level of the marker gene and/or the exogenous target gene in the descending order of expression levels.

d) returning the Nos. and location information of the screened high-protein-expression cell strains to the robot control software;

where for the cell Nos. and locations returned in step d, in this example, those of the top 100 cells ranked by predicted expression levels were returned, the Nos. are from 1 to 100, and the location information included coordinates in a planar coordinate system relative to the center of the microscope (the depth of the medium was not considered, as all selected cells settled at the bottom of the small dish. Additionally, during the photographing process, cells that did not settle at the bottom of the small dish may be out of focus, resulting in blurred cells, and cells with blurred images may also be excluded as candidate cells).

e) robotic control software automatically operating (or operating with manual assistance) the robotic arm to aspirate the screened monoclonal cell strain and transferring to the designated well plate, and repeating the process until all high-expression cell strains were transferred;

where for the process of the robotic arm aspirating the target monoclonal cell strain and transferring to the well plate, detail description was made in all of:

Patent Chinese patent literatures: "ROBOT-BASED CELL MANIPULATION TASK PROCESSING METHOD, APPARATUS, EQUIPMENT AND MEDIUM" with publication number CN113821287B, "ROBOT-BASED CELL ASPIRATION CONTROL METHOD, APPARATUS, EQUIPMENT, AND STORAGE MEDIUM" with publication number CN113821287B, "CONTROL METHOD, APPARATUS, EQUIPMENT, AND STORAGE MEDIUM FOR CELL MANIPULATION ROBOTS" with publication number CN113771030A, and "CONTROL INFORMATION CONFIGURATION METHOD, APPARATUS, EQUIPMENT, AND MEDIUM FOR CELL MANIPULATION ROBOTS" with publication number CN113733087B.

f) culturing the cell clones in the well plate to a certain quantity, then transferring to a larger well plate for further culturing, and finally expanding to shake flask culture, and detecting the cell expression level to determine that the cell strains selected by artificial intelligence were high-yielding cell strains;

where in this example, in step f, then were transferred to a 96-well plate for expansion culturing and then transferred to a shake flask for culturing.

(4) subculturing the monoclonal vector microparticle with high protein expression level obtained in step (3), determining whether the monoclonal vector microparticle had subculture stability based on the expression levels of the marker gene in multiple generations of vector microparticles to obtain the monoclonal vector microparticle with high expression level and subculture stability, and determining the insertion position of the exogenous gene on the endogenous gene as the site-directed integration site.

(4-1) acquiring the expression levels of the marker gene of multiple generations of the monoclonal vector microparticle; and

g) subculturing the screened high-yielding cell strains, where before the subculturing of each generation of cell strains, a part of the sample was diluted and placed in a culture dish and photographed by using a microscope, and the images of the cell strains of several generations were photographed continuously; and currently, by using photography, the expression situation of the selected cells may be predicted by the model subjected to the cell morphology learning, and the expression situation of the selected cells may also be determined by using fluorescent labeling.

For the monoclonal cell photographing in step g, in this example, a Thermo Fisher Scientific M7000 microscope was used to photograph the cells.

(4-2) evaluating the differences in the expression levels of the marker gene of multiple generations of monoclonal vector microparticles by using the intelligent algorithm according to the expression levels of the marker gene of the multiple generations of monoclonal vector microparticles obtained in step (4-1); and

(4-3) determining whether the monoclonal vector microparticle has subculture stability, according to the differences in expression levels of the marker gene of the multiple generations of monoclonal vector microparticles obtained in step (4-2).

h) inputting the collected images of several generations of the cell strain into an artificial intelligence algorithm to predict the subculture stability of the cell strain according to fluorescence intensity and/or protein expression level, selecting the cell strain having high protein expression property and stable subculturing as the final candidate cell strain, where the subculture stability of the cell strain may be predicted based on fluorescence, or cell morphology rather than fluorescence, the following step h involved predicting protein expression level based on cell morphology rather than fluorescence, and the final candidate cell strain was the site-directed integration vector microparticle.

The predicting the subculture stability of the cell strain in step h included but not limited to using traditional image recognition algorithm to extract image features by using histograms and perform prediction, using deep learning neural network to automatically extract image features and perform prediction, and other image-based prediction technologies. In this example, a deep learning-based method was used to automatically extract image features of stable cell strains and unstable cell strains and predict the stability, achieving an accuracy rate of the prediction results of 84% for different cell strains. This is described in detail in the Chinese patent document "CELL STRAIN STABILIYT PREDICTION METHOD, APPARATUS, COMPUTER DEVICE, AND STORAGE MEDIUM".

(5) expanding and culturing the stable cell strains screened by using AI to scale up to shake flask culture, and then monitoring the growth curve for 14 days, with CHO-K1 cells used as a control; inoculating under the inoculation density of $5 \times 10^5$/ml and the inoculation system of 30ml, counting the cells on the day of inoculation, the 3rd day of culture, the 5th day of culture, the 7th day of culture , the 9th day of culture, the 11th day of culture, the 13th day of culture, and the 14th day of culture, and screening and eliminating monoclonals with a growth rate lower than that of CHO-K1 to obtain the site-directed integration vector with high expression and the subculture stability higher than the CHO-K1 cell strain.

(6) performing stable subculture on the site-directed integration vector that exhibited high expression and had higher subculture stability than the CHO-K1 cell strain, with the subculture performed every 3 or 4 days, where the cell density of the 4-day subculture was $3 \times 10^5$/ml and the cell density of the 3-day subculture was $5 \times 10^5$/ml; monitoring the fluorescence value of the cell strain regularly during the subculture; performing a subculture stability study for about 90 days on the above-mentioned high-fluorescence cell strain by using the passage medium, where the cells with small fluorescence changes and stable cell strain growth were determined as stable high-fluorescence cells; and verifying the copy number and integration site of the stable high-fluorescence cells, where the stable high-fluorescence cells here were site-directed integration vectors with high subculture stability and high expression level.

The above-mentioned site-directed integration vector was used as an example to integrate the exogenous target gene and screened out the exogenous target gene expression vector microparticles with high target gene expression level and high subculture stability, and the site-directed integration method of the exogenous target gene provided by the present application included the following steps:

acquiring the site-directed integration vector microparticle provided by the present application, and performing homologous recombination by using the first homology arm in the exogenous gene in the site-directed integration vector microparticle, to recombine the exogenous target gene expression fragment into the gene of the site-directed integration vector microparticle and simultaneously remove the marker gene expression fragment.

The specific steps included:

(7) performing electroporating on the stable high-fluorescence cells by using a plasmid carrying an exogenous target gene, and co-transfecting with Bxb1 integrase into the stable high-fluorescence cells, where the plasmid carrying the exogenous target gene was an exogenous gene including two attB sequences and a target gene sequence between the two attB sequences; the exogenous gene included an exogenous target gene expression fragment that may replace the marker gene expression fragment inserted into the endogenous gene, the exogenous target gene expression fragment included two attB sequences and a target gene sequence between the two attB sequences; and under the action of Bxb1 integrase, the attP on the left side of the marker gene EGFP formed attL with the attB on the left side of the target gene, and the attP on the right side of the marker gene EGFP formed attR with the attB on the right side of the target gene, and simultaneously, the exogenous target gene expression fragment completed the replacement of the marker gene expression fragment.

Two days after electroporation, the plate was checked and the electroporation effect was recorded, and simultaneously hygromycin was used to perform pressurizing at a pressurizing concentration of 250ug/ml, then the cells were aliquoted into 96-well plate at 6,000-10,000 cells per well.

The 96-well plate was placed in a carbon dioxide constant-temperature incubator (37°C, 80% humidity) and cultured for 1-2 weeks, the proportion of non-fluorescent cells in the minipool was quantified, the non-fluorescent cells was manually enriched, and the monoclonalization was performed by limiting dilution.

(8) placing the monoclonal 96-well plate in a carbon dioxide constant-temperature incubator (37°C, 80% humidity) and culturing for 14 days, during which monoclonal cells were photographed on the day of inoculation, the first day of culture, the second day of culture, the third day of culture, the seventh day of culture, and the fourteenth day of culture to confirm the status of monoclonal cells and fluorescence.

The formation of monoclonal cells and the methods for screening stable high-expression cell strains may also be achieved through artificial intelligence, and the specific steps of artificial intelligence implementation were as described in the a-h processes in step 5 above, with the difference that in this step c, high expression prediction analysis was performed by using a prediction model generated by performing artificial intelligence image learning on historical images of CHO cells including marker gene expression fragments with a length similar to the DNA sequence length of the exogenous target gene expression fragment inserted into the target cell strain, thereby obtaining exogenous target gene expression vector microparticles.

(9) after 14 days, expanding the confirmed non-fluorescent and normally growing monoclonals to a 24-well plate, placing and culturing in a constant-temperature incubator (37°C, 80% humidity), continuously expanding to a shake flask stage for culturing, where after approximately 1 month, the cell strains were subjected to fed-batch culture (Advance+1% Glutamax, i.e. the fed-batch medium, for the first three days), 3% (v/v) Cell Boost 7a and 0.3% (v/v) Cell Boost 7b (cytiva) were added at the same time on the 3rd day of the culture and the 5th day of the culture, 5% (v/v) Cell Boost 7a and 0.5% (v/v) Cell Boost 7b (cytiva) were added at the same time on each of the 7th day of the culture, the 9th day of the culture, the 11th day of the culture and the 13th day of the culture, and the sugar was replenished according to the sugar consumption of cells and the expression level was evaluated on the 3rd day of the culture, the 5th day of the culture, the 7th day of the culture, the 9th day of the culture, the 11th day of the culture and the 13th day of the culture.

(10) confirming that the monoclonal cells with relatively high expression were continuously passaged for approximately 90 days, during which $5 \times 10^6$ cells may be retained to extract the genome for site replacement verification and residual gene detection; and then, performing batch culture on the 1st, 13th and 26th generations for 7 days to confirm expression level and the stability of QP, where "the stability of QP" refers to the stability of the daily production of a single cell;

Steps 1-6 in the above process completed the "parental cell line construction + screening", completing the parental cell line construction and screening through a one-step method and obtaining site-directed integration vector microparticles; step 7 and step 8 in the above process completed the "target cell line construction + first screening", and achieved site-directed integration of the exogenous target gene, completing the initial screening of the target cell strain; the target cell strains obtained by the initially screening were then screened a second time and a third time in step 9 and step 10 to screen out exogenous target gene expression vector microparticles with higher expression level and better stability. The insertion position of the exogenous gene where the exogenous target gene expression fragment was located in the exogenous target gene expression vector microparticle is the same as that of the exogenous gene where the marker gene expression fragment was located in the site-directed integration vector microparticle; and when the same site-directed integration vector microparticle was used, the insertion positions of the exogenous genes where the exogenous target gene expression fragments were located in the exogenous target gene expression vector microparticles obtained by the aforementioned exogenous target gene integration method were the same, thereby achieving site-directed integration of the target gene.

The two-step screening method of "parental cell strain construction + screening" used in the prior art requires 12 months. The one-step screening which uses steps 1-6 in the above process to complete the "parental cell strain construction + screening" requires only 6 months, which is 50% shorter in time, and the parental cell strain is highly stable and has high expression level. The main reason for the significant time reduction is the use of the one-step method of "parental cell strain construction + screening" and the efficient and rapid screening of monoclonal cell strains through artificial intelligence.

In the prior art, completing the "target cell strain construction + screening" requires 12 months. By adopting step 7- step 10 in the above process to complete the "target cell strain construction + screening", only 6 months are required, thus the time is shortened by 50% and the target cell strain is highly stable and has high expression level of the exogenous target gene. This significant time reduction is primarily due to that the parental cell strain has high stability and high expression level and the exogenous target gene is inserted into a specific site in the parental cell strain through site-directed integration, thereby shortening the "target cell strain construction + screening" process; and the prepared target cell strain is highly stable and has high expression level of the exogenous target gene.

Furthermore, when predicting the expression level, images of cells into which the exogenous gene is inserted are used for learning; when predicting parental cells, historical cell images of original cells into which the exogenous gene having the length similar to that of the DNA sequence of the exogenous gene inserted into the parental cells are inserted are used for learning, for early prediction of expression levels and then screening of parental cell strains. When predicting target cell strains, cell images of parental cells into which the exogenous gene having the length similar to that of the DNA sequence of the exogenous gene are inserted are used for learning, for early prediction of expression levels and then screening of product cell strains, thereby increasing the probability of obtaining sites that may highly express exogenous target genes. Such arrangement may reduce the occurrence of situations where the exogenous target gene may not be highly expressed due to a significant difference in length between the exogenous target gene expression fragment containing the exogenous target gene that needs to be site-specifically integrated into a specific site and the marker gene expression fragment inserted during the screening of parental cells.

Example 2: Screening of S6C7 site and preparation of site-directed integration vector microparticle and exogenous target gene expression vector microparticle thereof

This example prepared the site-directed integration vector microparticle and the exogenous target gene expression vector microparticle according to the method described in Example 1, with the differences from Example 1 as follows.

The specific distinguishing steps included that:

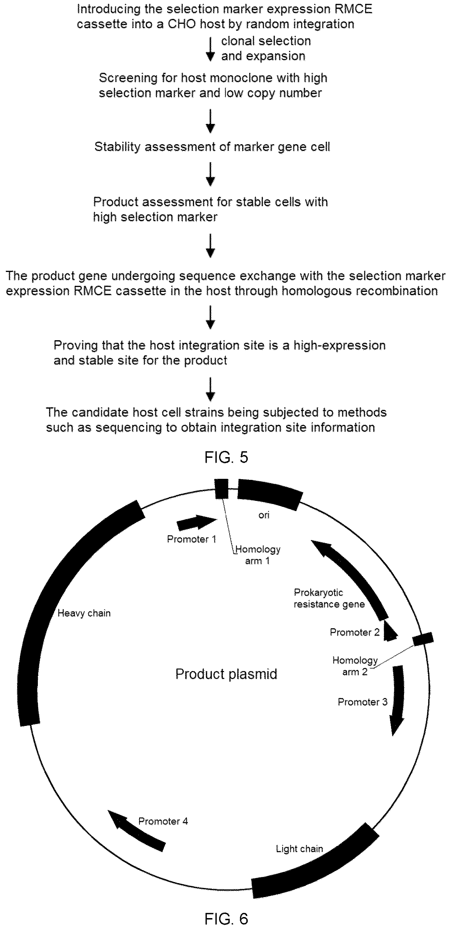

(1) the operation flow of Example 2 was as shown in FIG. 5; specific consumables involved: Neon Resuspension Buffer R (ThermoFisher), which was a resuspension buffer dedicated for cell electroporation instrument; E1 Buffer (ThermoFisher), which was an electroporation buffer; a recovery medium, which was prepared by adding 1% GlutaMAX (ThermoFisher) to 80% (v/v) EX-CELL CHO Cloning Medium (Sigma-Aldrich) and 20% (v/v) EX-CELL Advanced CHO Fed-batch Medium (Sigma-Aldrich); an expansion medium and a passage medium, which were both prepared by adding 1% GlutaMAX (ThermoFisher) to EX-CELL Advanced CHO Fed-batch Medium; a pressurized medium 1, which was prepared by adding G418 to the passage medium to a final concentration of 800μg/ml; a pressurized medium 2, which was prepared by adding Hygromycin to the passage medium to a final concentration of 250μg/ml; a conditioned medium, which was the supernatant obtained by inoculating CHO-K1 in the passage medium, culturing for 1 day, and then performing sterile filtration; a cloning medium, which was prepared by adding 1% GlutaMAX to 75% (v/v) EX-CELL CHO Cloning Medium, 20% (v/v) conditioned medium, and 5% (v/v) ClonaCell-CHO ACF Supplement; and in fed-batch medium, a basal medium which was prepared by adding 1% GlutaMAX (ThermoFisher) to EX-CELL Advanced CHO Fed-batch Medium, and the feed medium, which was Cell Boost 7a/7b (HyClone);

(2) The site-directed integration vector microparticle obtained in step 6 was named GBB003 parental cell.

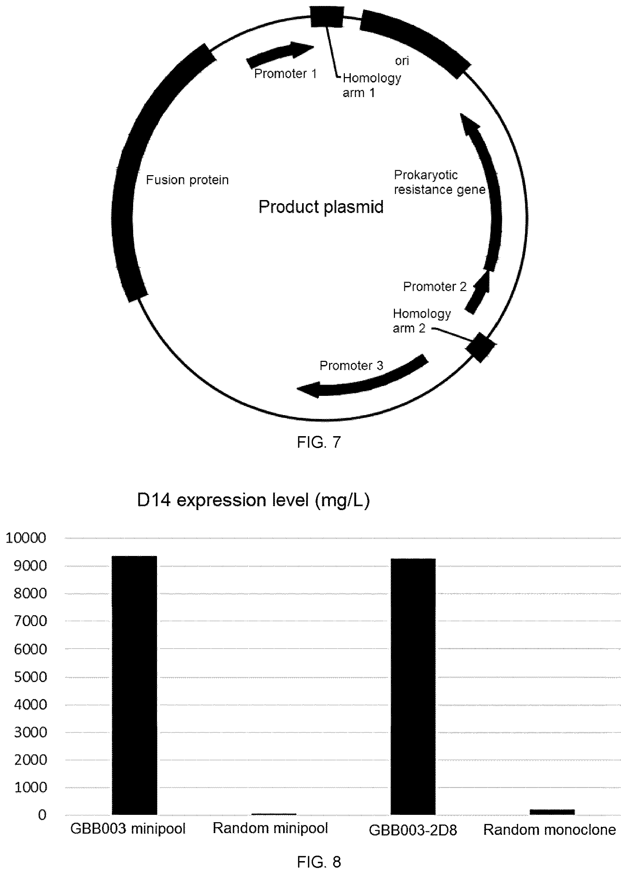

(3) The target genes in step 8 were target gene 1 (monoclonal antibody), target gene 2 (monoclonal antibody), target gene 3 (fusion protein), target gene 4 (bispecific antibody; IgG-SCFV), and target gene 5 (bispecific antibody; KiH-Crossmab) respectively. The target gene 1 to the target gene 5 were respectively cloned into different plasmids by means of restriction enzyme digestion and ligation to construct recombinant plasmids containing RMCE. The recombinant plasmid of the target gene 1, the recombinant plasmid of the target gene 2, the recombinant plasmid of the target gene 4, and the recombinant plasmid of the target gene 5 are as shown in FIG. 6, and the recombinant plasmid of the target gene 3 is as shown in FIG. 7.

In GBB003 parental cells, the length of the exogenous fragment inserted into the endogenous gene was 7545 bp, the length of the marker gene expression fragment was 3815 bp, and the length of the remaining fragment was 3730 bp. Among them, the length of the exogenous target gene expression fragment corresponding to the target gene 1 was 6817 bp, the length of the exogenous target gene expression fragment corresponding to the target gene 2 was 6866 bp, the length of the exogenous target gene expression fragment corresponding to the target gene 3 was 2548 bp, the length of the exogenous target gene expression fragment corresponding to the target gene 4 was 7688 bp, and the length of the exogenous target gene expression fragment corresponding to the target gene 5 was 12256 bp; and the stability of the site and/or the editable length of the gene fragment was verified by inserting gene fragments of different lengths.

The exogenous target gene 1 had a light chain sequence as shown in SEQ ID No. 2; and the exogenous target gene 1 had a heavy chain sequence as shown in SEQ ID No. 3;

the exogenous target gene 2 had a light chain sequence as shown in SEQ ID No. 4; and the exogenous target gene 2 had a heavy chain sequence as shown in SEQ ID No. 5;
the exogenous target gene 3 had the sequence as shown in SEQ ID No. 24;
the exogenous target gene 4 had a light chain sequence as shown in SEQ ID No. 25; and the exogenous target gene 4 had a heavy chain sequence as shown in SEQ ID No. 26; and
the exogenous target gene 5 had the light chain 1 sequence of the target gene 5 as shown in SEQ ID No.27, the heavy chain 1 sequence of the target gene 5 as shown in SEQ ID No.28, the light chain 2 sequence of the target gene 5 as shown in SEQ ID No.29 and the heavy chain 2 sequence of the target gene 5 as shown in SEQ ID No.30.

(4) In step 9, the comparison results of the expression levels of the site-specifically integrated product minipool cell pool (GBB003-minipool), the exogenous target gene 1 expression vector microparticle (denoted as GBB003-2D8 cell), and the randomly integrated minipool cell pool (random minipool) and the single-copy cell strain (random monoclonal clone) cultured under the same conditions are as shown in FIG. 8.

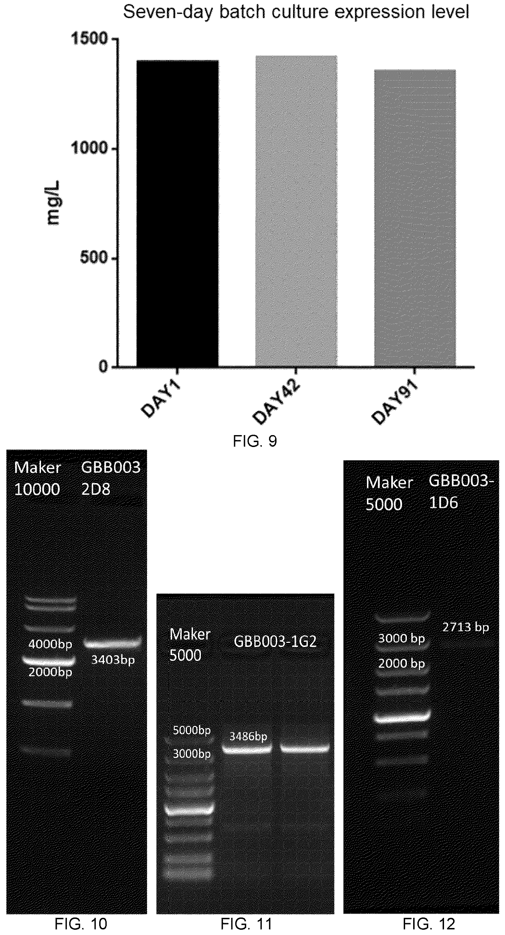

(5) In step 10, the 1st, 13th, and 26th generations were batch cultured for 7 days respectively, sugar was replenished on the 3rd, 5th, and 7th days according to the sugar consumption of the cells, the expression levels are as shown in FIG. 9, and the expression levels show that the monoclonal cells are stable and highly productive.

The parental cell GBB003, corresponding to the cell strain with high expression of both the fluorescent gene and the target gene, was subjected to next-generation whole-genome sequencing; it was revealed that the parental cell GBB003 was a single-copy, and the annotation information of the integration site on CHO-K1 was: NW_003616785.1:83044; and the integration site was the insertion position of the exogenous gene in the endogenous gene, and the integration site was named S6C7.

The sequence fragment after integration of the target gene 1 was as shown in SEQ ID No. 6; the genome of the GBB003-2D8 cell was amplified using the breakpoint forward primer F1 (SEQ ID No. 7): AGACCAGCCTCAGATGTC ACAC and the reverse primer R1 (SEQ ID No. 8): AGGCACACAACGGAGGCGGT of the target gene on the plasmid used for integration; and the reverse primer R1 of the target gene 1 was located in the light chain of target gene 1, and a fragment with a theoretical size of 3403 bp was obtained, as shown in FIG. 10 (band corresponding to the GBB003-2D8 cell lane).

In the above, the sequence fragment after integration of the target gene 1 included attL, the nucleotide sequence of the attL was as shown in SEQ ID No. 9, where SEQ ID No. 9: ATGATCCTGACGACGGAGACCGCGGTGGTTGACCAGACAAACC; in the sequence fragment after integration of the target gene 1, the part before the attL was an exogenous sequence, while the part thereafter was an endogenous sequence. The exogenous sequence included a part of the sequence of the light chain reading frame of the target gene 1, namely, the sequence amplified from SEQ ID No. 8; and the target gene 1 may encode a monoclonal antibody.

The amplified band was cut and recovered from the gel, and purified, and multiple pairs of primers were designed for DNA sequencing; and after splicing the sequencing results, the sequence was as shown in SEQ ID No. 6, which was consistent with the theoretical sequence, thus confirming that the target gene 1 was correctly integrated into the corresponding position of the marker gene expression fragment.

Further, it showed that the vector microparticle CHO-K1 cell had an S6C7 site, which was located within the fragment SEQ ID No. 1 as shown in SEQ ID No. 1; and specifically, the annotation information of the integration site S6C7 on CHO-K1 was: NW_003616785.1:83044.

Similarly, the sequence fragment after site-directed integration of the exogenous target gene 2 into GBB003 parental cell as was shown in SEQ ID No. 10.

The genome of the cell strain obtained by site-specifically integrating the exogenous target gene 2 into GBB003 parental cell was amplified using the above-mentioned breakpoint forward primer F1 (SEQ ID No. 7): AGACCAGCCT CAGATGTCACAC and the target gene 2 reverse primer R2 (SEQ ID No. 11): CCTTAGAATCCTGCTCGGTGA on the inserted fragment of the exogenous target gene 2, to obtain a fragment with the theoretical size (3486 bp), as shown in FIG. 11 (band corresponding to the GBB003-1G2 lane).

The amplified band was cut and recovered from the gel, and purified, and multiple pairs of primers were designed for DNA sequencing; after splicing the sequencing results, the sequence was as shown in SEQ ID No. 10, which was consistent with the theoretical sequence (SEQ ID No. 10), thus confirming that the exogenous target gene 2 was correctly integrated into the corresponding position of the marker gene expression fragment. In the above, the sequence fragment after integration of the exogenous target gene 2 contained attL, and in the sequence fragment after integration of the exogenous target gene 2, the part before attL was the exogenous sequence, and the part thereafter was the endogenous sequence. The exogenous sequence included the target gene 2.

Similarly, the sequence fragment after site-directed integration of the exogenous target gene 3 into GBB003 parental cell was as shown in SEQ ID No. 31; the genome of the cell strain obtained after site-specifically integrating the exogenous target gene 3 into GBB003 parental cell was amplified using the above breakpoint forward primer F1 (SEQ ID No. 7): AGACCAGCCTCAGATGTCACAC and the target gene 3 reverse primer R2 (SEQ ID No. 32): CTCGACGCCA TCCACGTACCAG on the inserted fragment of the exogenous target gene, to obtain a fragment with the theoretical size (2713 bp), as shown in FIG. 12 (band corresponding to the GBB003-1D6 lane), thus confirming that the exogenous target gene 3 was correctly integrated into the corresponding position of the marker gene expression fragment. In the above, the sequence fragment after integration of the exogenous target gene 3 contained attL, and in the sequence fragment after integration of the exogenous target gene 3, the part before attL was the exogenous sequence, and the part thereafter was the endogenous sequence. The exogenous sequence included the target gene 3.

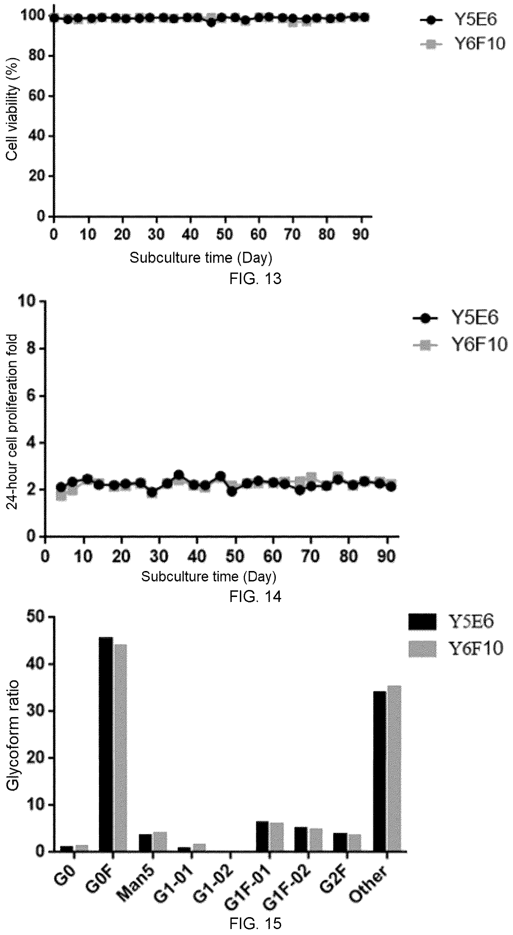

Similarly, after the target gene 4 was integrated into GBB003 parental cells, two cell strains, Y5E6 and Y6F10, were selected through AI prediction as exogenous target gene expression vector microparticles, which were subjected to 90-day subculture. The subcultured cell viability stabilities are as shown in FIG. 13, and the cell proliferation rate stabilities are as shown in FIG. 14.

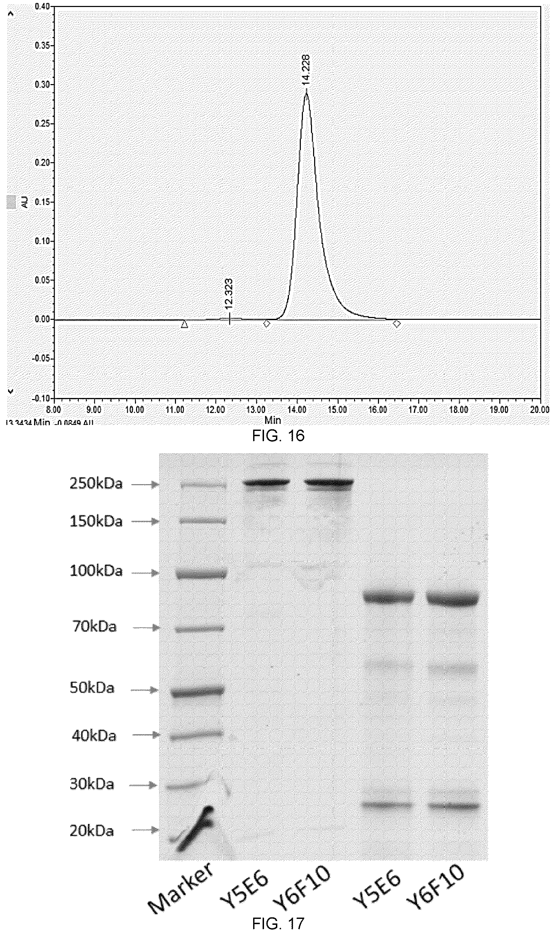

The results of glycoforms, product quality attributes, of the Y5E6 cell and the Y6F10 cell formed after integration are as shown in FIG. 15, the charge variation comparison results are as shown in FIG. 16, the results of sodium dodecyl sulfate-polyacrylamide gel electrophoresis detection are as shown in FIG. 17. The above results demonstrate that the GBB003 parental cell may be integrated with an exogenous target gene expression fragment with a length of 7688 bp and may stably express the target gene in the exogenous target gene expression fragment with a length of 7688 bp.

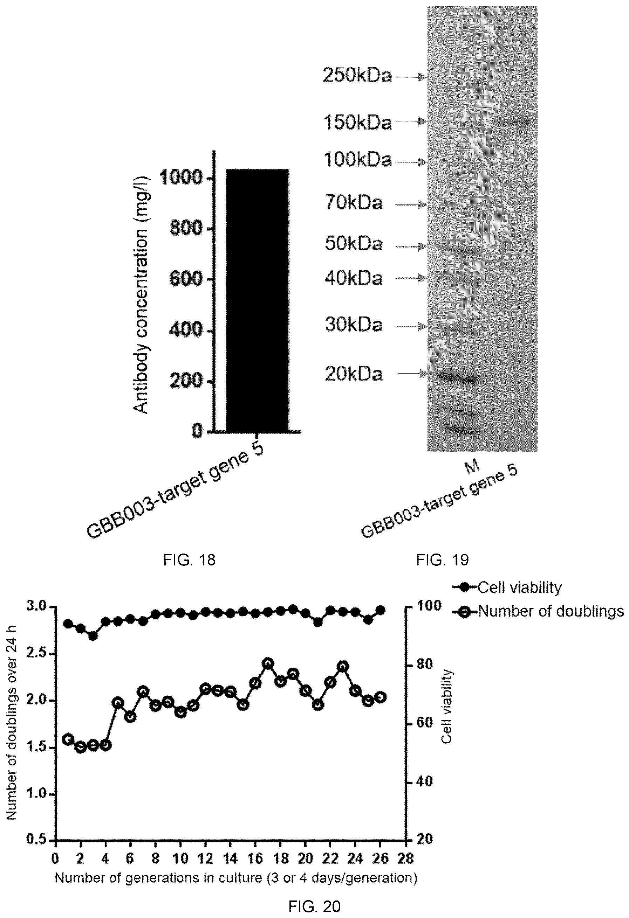

Similarly, the concentration results of the antibody expressed by target gene 5 after 6-well batch culture of the cell pool obtained after target gene 5 was integrated into GBB003 parental cells are as shown in FIG. 18, and the results of sodium dodecyl sulfate-polyacrylamide gel electrophoresis detection are as shown in FIG. 19. The above results demonstrate that the GBB003 parental cell strain may be integrate with the exogenous target gene expression fragment of a length of 12256 bp and may stably express the target gene in the exogenous target gene expression fragment of a length of 12256bp.

Combined with the expression and subculture stability of the GBB003 parental cell strain integrated with the target gene 1, target gene 2, target gene 3, target gene 4, or target gene 5, it is found that the site-directed integration vector microparticles screened out by the method of this example may be used to integrate exogenous target gene expression fragments of different lengths.

Example 3: Screening of S4F6 sites and preparation of site-directed integration vector microparticle and exogenous target gene expression vector microparticle thereof

In this example, the screening of the S4F6 site and the preparation of its site-specific integration vector microparticle and the exogenous target gene expression vector microparticle thereof were carried out according to the solution of Example 2. The differences from Example 2 were as follows.

The specific distinguishing steps included that:

(1) the consumables in the specific embodiment involved: Neon Resuspension Buffer R (ThermoFisher), which was a resuspension buffer dedicated for cell electroporation instrument; E (ThermoFisher), which was an electroporation buffer; EX-CELL Advanced CHO Fed-batch Medium (Sigma-Aldrich), which was a recovery medium; an expansion medium, which was prepared by adding 200 $\mu$g/ml hygromycin (ThermoFisher) and 1% GlutaMAX (ThermoFisher) to EX-CELL Advanced CHO Fed-batch Medium; a shake flask medium, which was prepared by adding 1% GlutaMAX to EX-CELL Advanced CHO Fed-batch Medium; and in fed-batch medium, a basal medium which was 100% EX-CELL Advanced CHO Fed-batch Medium, and feed medium, which was 4% Cell Boost 7a/7b (HyClone).

(2) In step 6, the high-fluorescence cell strain was subjected to a subculture stability study for approximately 90 days, with subculture performed every 3 or 4 days; the cell density for 4-day subculture was $3\times10^5$ cells/ml, and the cell density for 3-day subculture was $5\times10^5$ cells/ml; during the subculture, the fluorescence value of the cell strain was monitored, and cells with minimal changes in fluorescence value and stable cell strain growth were identified as stable high-fluorescence cells, and this cell strain was named GBB-01 cell; the GBB-01 cell was a parental cell strain and the GBB-01 cell was a preferred site-directed integration vector microparticle. FIG. 19 shows that the GBB-01 cells maintained stable growth during the 90-day subculture, and Table 1 shows that the GBB-01 cells exhibited stable protein expression.

Table 1

|  | Mean fluorescence value | Fluorescent cell percentage (%) |
|---|---|---|
| 3rd generation (the 10th day) | 25.5 | 100 |
| 26th generation (the 91st day) | 22.3 | 100 |

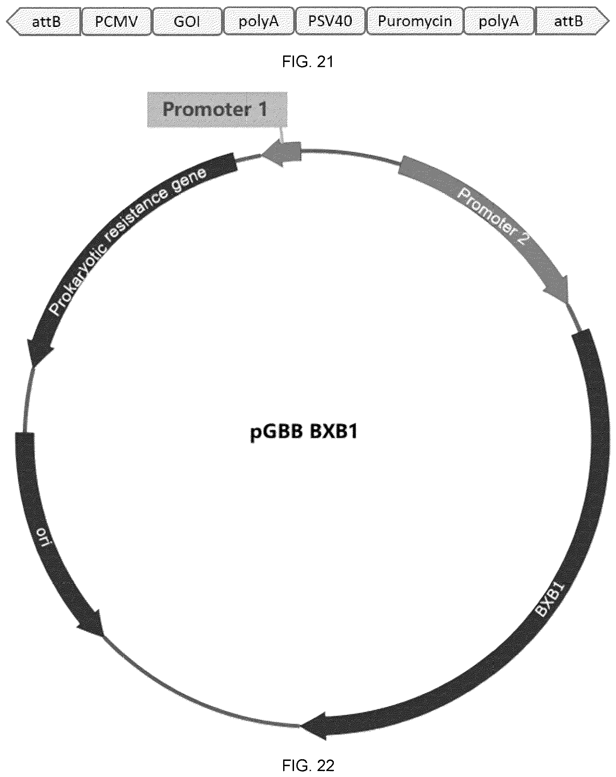

(3) In step 7, the recombinant plasmid of target gene 6 contained an exogenous target gene 6 expression fragment with the target gene 6 between the attBs. The target gene 6 fragment was used to be cloned into the exogenous target gene 6 expression fragment (as shown in FIG. 21 ) for electroporation verification, and co-transfected with the Bxb1 integrase plasmid (as shown in FIG. 22 ) into the parental cell strain, where the prokaryotic resistance gene was used for prokaryotic plasmid construction and screening, the homology arm 1 (attP in the marker gene expression fragment in the parental cell GBB-01) and homology arm 2 (attB in the exogenous target gene expression fragment) were used to produce recombination replacement with the screened parental cell strain, the promoter 1 was used to drive the expression of the hygromycin gene after recombination with the parental cell, and the promoter 3 was used to drive the expression of the target gene. BamH I and Xho I enzymes were used to achieve restriction enzyme digestion and insertion. The structure of Bxb1 integrase plasmid is as shown in FIG. 22, where the prokaryotic resistance gene is used for prokaryotic plasmid construction and screening, the promoter 1 is used to drive the expression of the Bxb1 recombinase gene, and the promoter 2 is used to drive the expression of the prokaryotic resistance gene.

The length of the exogenous gene fragment inserted into the endogenous gene in GBB-01 parental cells was 7545 bp, the length of the marker gene expression fragment in the exogenous gene was 3815 bp, and the length of the remaining fragment of the exogenous gene is 3730 bp. The length of the exogenous target gene expression fragment containing the target gene 6 was 2227 bp.

The target gene 6 fragment had the sequence as shown in SEQ ID No.13.

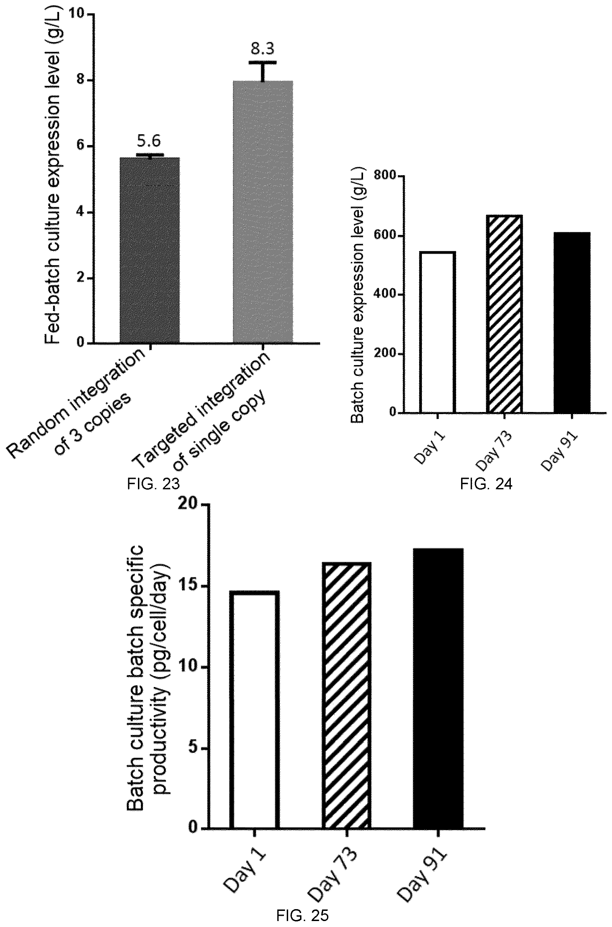

(4) In step 9, the non-fluorescent cells in the monoclonal cells (i.e., the expression vector microparticles of the exogenous target gene 6) were further expanded (using the expansion medium), and expanded to the shake flask stage for culturing (using the shake flask medium), and the expression level was evaluated by fed-batch culture (using the fed-batch medium). The expression level comparison result of the expression vector microparticle of the exogenous target gene 6 site-specifically integrated (denoted as GBB-01-H8 cells) and the cell strain with three randomly integrated copies of the target gene 6 cultured under the same conditions is as shown in FIG. 23, where "single targetedly integrated copy" refers to GBB-01-H8 cell.

(5) In step 10, GBB-01-H8 cells were continuously subcultured for approximately 90 days for stability assessment, followed by 7 days of batch culture (cultured in basal medium only). The results showed that GBB-01-H8 cells exhibited subculture stability and high expression level, both in terms of expression level (FIG. 24) and specific productivity (FIG. 25).

The quality attributes of GBB-01-H8 cells were studied and compared with the expression products of cell strains obtained by random integration method in the following aspects:

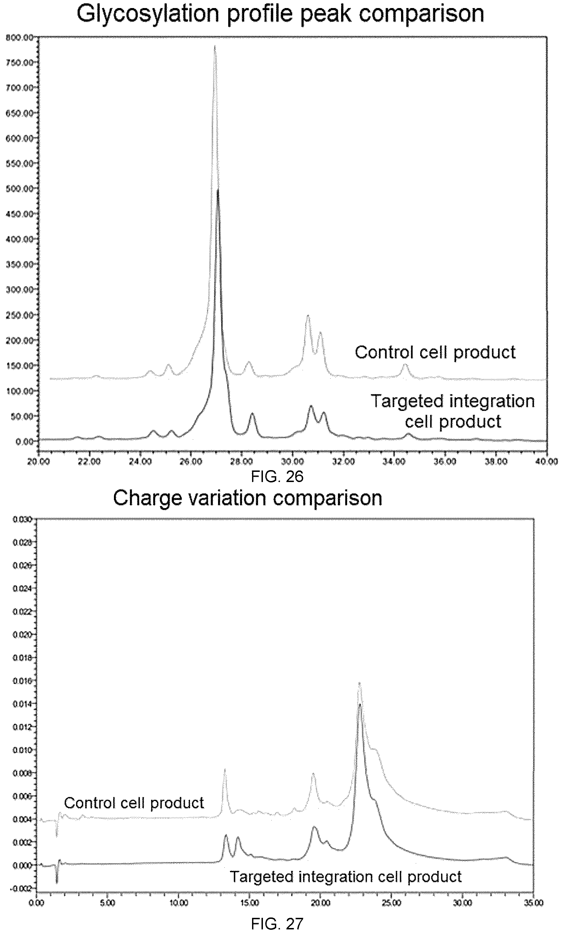

according to the glycosylation profile comparison result in FIG. 26 and the charge variation comparison results in FIG. 27, the results showed that the quality attributes of the targeted integration cell product (i.e., GBB-01-H8 cell) and the control cell product (i.e., the cell strain expression product obtained by the random integration method) were similar, with no significant differences.

The GBB-01 cells corresponding to GBB-01-H8 cells with high expression of both fluorescent protein EGFP and target gene 6 were subjected to next-generation whole-genome sequencing, and the annotation information of the integration site on CHO-K1 was acquired as follows: NC_048596:34375010, which was located in the intergenic region. This integration site was the insertion position of the exogenous gene in the endogenous gene, and the integration site was named S4F6.

NC_048596 sequence fragment after integration of the target gene 6 is as shown in SEQ ID No.14.

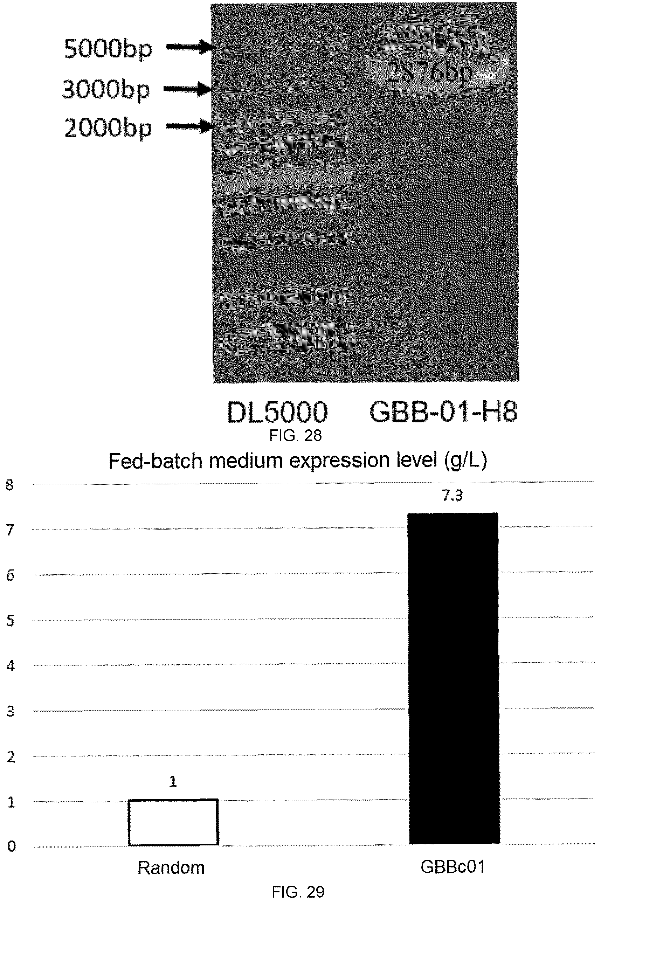

GBB-01-H8 cell genome was amplified using the above-mentioned breakpoint forward primer F (SEQ ID No. 15): tgcagccacactaagatgg and the target gene 6 reverse primer R (SEQ ID No. 16): ccagagacaggctcaaggac on the exogenous target gene 6 expression fragment, to obtain a fragment with the theoretical size (2876 bp), as shown in FIG. 28 (band corresponding to the GBB-01-H8 lane).

The amplified band was cut and recovered from the gel, and purified, and multiple pairs of primers were designed for DNA sequencing; and after splicing the sequencing results, the sequence was as shown in SEQ ID No. 14, which was consistent with the theoretical sequence, thus confirming that the target gene 6 was correctly integrated into the corresponding position of the marker gene expression fragment.

Comparative Example 1. Performance comparison of the exogenous target gene expression vector microparticle prepared by the method of the present application and the exogenous target gene expression vector microparticle constructed by CRISPR/Cas9

(1) Construction of Cas9 editing plasmid:
a sgRNA capable of targeting the base interval from positions 1678 to 1744 of the nucleic acid fragment as shown in SEQ ID No.12, having high cleavage efficiency and having a sequence as shown in SEQ ID No. 17 (SEQ ID No. 17: gatagtcacccacaccagaggttttagagctagaaatagcaagttaaaataaggctagtccgttatcaacttgaaaaagtggcaccgagtcggtgc; GC content = 55%, no secondary structure) was designed, and the double strands were combined and annealed. The reaction system consisted of $5\mu L$ each of forward and reverse oligos, $5\mu L$ of $10\times$ NEB buffer 2, and was adjusted to a total volume of $50\ \mu L$ with water. The system was incubated at 95°C (must be measured with a thermometer) for 5 min, and then cooled down to 25°C naturally. The obtained product plasmid was ligated into the pDonor CRISPR/Cas9 vector digested with Pvul enzyme. The ligation product was transformed into DH5$\alpha$ competent cells and plated onto ampicillin-resistant LB plate. The next day, single colonies were picked and the plasmid was extracted. Finally, the correct Cas9 encoding plasmid was obtained through verification by first-generation sequencing.

(2) Construction of donor plasmid:
the donor plasmid consisted of a 5' homology arm, an anti-PD1 monoclonal antibody encoding gene, a hygromycin encoding gene and a 3' homology arm connected in series on a pUC19 plasmid.

(3) Electroporation transfection of Cas9 encoding plasmid and anti-PD1 donor plasmid:
suspension Chinese hamster ovary cancer cell, namely the CHO-K1 cell strain, were used. The day before transfection, the cell density was adjusted to $5\times10^5$ cells /mL. The next day, transfection was performed by using electroporation under conditions of a voltage of 1575V, a pulse duration of 10ms, and three pulses. A 6-well cell culture plate was used for transfection, with $3\times10^6$ cells, 2mL of medium, and $15\mu g$ of plasmid required. After transfection, the cells were cultured in a 37°C, 5% $CO_2$ incubator. In the above, co-transfection with the Cas9 encoding plasmid and the anti-PD1 donor plasmid resulted in a site-specifically integrated cell pool, while transfection with the anti-PD1 donor plasmid alone resulted in a random integrated cell pool.

(4) The site-specifically integrated cell pool was subjected to pressurized screening and enrichment.

(5) The site-specifically integrated cell pool was preliminarily identified by 5'/3' junction PCR, the target fragment obtained by amplification was sequenced, and the supernatant was collected after determining that the site-specifically integrated cell pool is obtained.

(6) Western blot was used to detect the expression of anti-PD1 antibodies in the site-specifically integrated cell pool, confirming that the site-specifically integrated cell pool was obtained. After 3 weeks of drug screening, the expression of anti-PD1 antibodies in the randomly integrated cell pool was basically undetectable by Western blot, while the expression level of anti-PD1 antibodies in the site-specifically integrated cell pool was higher.

(7) Obtaining monoclonal cell strains by limiting dilution method: monoclonal cell strains were obtained by the limiting dilution method using the previously site-specifically integrated cell pool. The cell pool was plated on a 96-well plate with a cell density of 0.8 cells/well. The plate was checked on the same day to confirm the monoclonality. When the cells grew for about 5 weeks, $1\times10^6$ monoclonal cells were aspirated and genomic DNA was extracted. 5'/3' junction PCR was performed again using the same method as step (5). The positive cell strains obtained by junction PCR were further subjected to Western blot detection to determine whether the anti-PD1 antibody was correctly expressed.

(8) Evaluation of the expression level of anti-PD1 antibodies of site-specifically integrated cell strains: the expression level of anti-PD1 antibodies of the site-specifically integrated cell strains was approximately 2.3 times that of randomly integrated cell strains. The site-specifically integrated cell strains may also be understood as the targetedly integrated cell strains, where the targeted site was a fixed site.

Table 2

| Integration site | Anti-PD1 antibody expression level of targetedly integrated cell strains (mg/L) |
|---|---|
| NC_048596:34375010 | 705 |
| Cell strain with single randomly integrated copy | 285mg/L |

According to the steps in Example 3, exogenous target gene expression vector microparticles for GBB-01 cells were constructed, using the anti-PD1 antibody editing gene as the target gene and the S4F6 site (NC_048596:34375010) as the site-directed integration site. A cell strain with a single randomly integrated copy of the anti-PD1 antibody editing gene was used as a control. The expression levels in 6-well plate batch culture in basal medium are as shown in Table 2, where the anti-PD1 antibody expression level of the cell strain with a single randomly integrated copy in the 6-well plate batch culture was 285 mg/L, while the expression level of the exogenous target gene expression vector microparticles for GBB-01 cells with the anti-PD1 antibody editing gene as the target gene and the S4F6 site as the site-directed integration site was 705 mg/L, which was 2.47 times the expression level of the cell line with a single randomly integrated copy, and was superior to the exogenous target gene expression vector microparticles constructed with CRISPR/Cas9.

Example 4: Screening of S2C4 site and preparation of site-directed integration vector microparticle and exogenous target gene expression vector microparticle thereof

In this example, the screening of the S2C4 site and preparation of its site-directed integration vector microparticle and the exogenous target gene expression vector microparticle were carried out according to the solution of Example 2. The difference from Example 2 was as follows.

The specific distinguishing steps included that:

(1) the consumables in the specific embodiment involved: Neon Resuspension Buffer R (ThermoFisher), which was a resuspension buffer dedicated for cell electroporation instrument; E (ThermoFisher), which was an electroporation buffer; a recovery medium, which was EX-CELL Advanced CHO Fed-batch Medium (Sigma-Aldrich); an expansion medium, which was prepared by adding 200 μg/ml hygromycin (ThermoFisher) and 1% GlutaMAX (ThermoFisher) to EX-CELL Advanced CHO Fed-batch Medium; a shake flask medium, which was prepared by adding 1% GlutaMAX to EX-CELL Advanced CHO Fed-batch Medium; and in fed-batch medium, a basal medium which was prepared by adding 1% GlutaMAX to 100% EX-CELL Advanced CHO Fed-batch Medium, and a feed medium, which was 4% Cell Boost 7a/7b (HyClone).

(2) In step 1, cells in the stable cell pool were screened for highly fluorescent cells, to acquire the top 1% of cells with the highest fluorescence.

(3) In step 2, the growth situation of the enriched cells was observed at any time. When the confluency reached 50% or above, the cells were expanded, following the scale-up sequence from 96-well plates to 6-well plates.

(4) In step 6, the monoclonal fluorescent site-directed integration vector microparticles for insertion of the marker gene expression fragment into the endogenous gene were amplified and cultured, and subjected to stable subculture, with the subculture performed every 3 or 4 days, where the cell density was $3 \times 10^5$ cells/ml for 4-day subculture and $5 \times 10^5$ cells/ml for 3-day subculture. The fluorescence value of the cell strain was monitored during the subculture, with subculture lasting for a total of 90 days. The highly fluorescent cell strain was retained as the preferred site-directed integration vector microparticle, recorded as GBBc001 cell, and used as the parental cell for subsequent site-directed integration of the exogenous target gene.

(5) In step 7, the target gene 3 (fusion protein) or target gene 1 (monoclonal antibody) fragment was cloned into the plasmid to construct a target gene 3 recombinant plasmid containing RMCE (as shown in FIG. 7) and a target gene 1 recombinant plasmid containing RMCE (as shown in FIG. 6). The target gene 3 recombinant plasmid contained the exogenous target gene 3 expression fragment with the target gene 3 between attBs. The target gene 1 recombinant plasmid contained the exogenous target gene 1 expression fragment with the target gene 1 between attBs.

(6) In step 8, the proportion of non-fluorescent cells in the cell pool was quantified, and after the cell viability recovered to 90% or above, the cells were enriched and expanded in culture. After the viability recovered to 90%, the cells were plated using the limiting dilution method to monoclonalize the cell pool.

(7) In step 9, after 14 days, the confirmed non-fluorescent and normally growing monoclonal exogenous target gene expression vector microparticles (i.e., monoclonal cells) were expanded to a 24-well plate for further expansion (Advanced+1%Glutamax), and then expanded to the shake flask stage culture (Advanced+1%Glutamax). After about 1 month, the cell strains were subjected to fed-batch culture, (Advance+1%Glutamax, for the first three days, 3% 7a and 0.3% 7b (cytiva) added on the 3rd and 5th days, 5% 7a and 0.5% 7b (cytiva) added on each of the 7th day of the culture, the 9th day of the culture, the 11th day of the culture and the 13th day of the culture), and the sugar was replenished according to sugar consumption of cells and the expression levels were evaluated on the 3rd day of the culture, the 5th day of the culture, the 7th day of the culture, the 9th day of the culture, the 11th day of the culture and the 13th day of the culture. The expression level comparison result of the exogenous target gene 3 expression vector microparticle with a single site-specifically integrated copy of the target gene 3 and randomly integrated cell strain cultured under the same conditions is as shown in FIG. 29 (GBBc001 in this figure represents the exogenous target gene 3 expression vector microparticle). The expression level comparison result of the exogenous target gene 1 expression vector microparticle with a single site-specifically integrated copy of the target gene 1 and randomly integrated cell strain cultured under the same conditions is as shown in FIG. 29 (GBBc001 in this figure represents the exogenous target gene 1 expression vector microparticle).

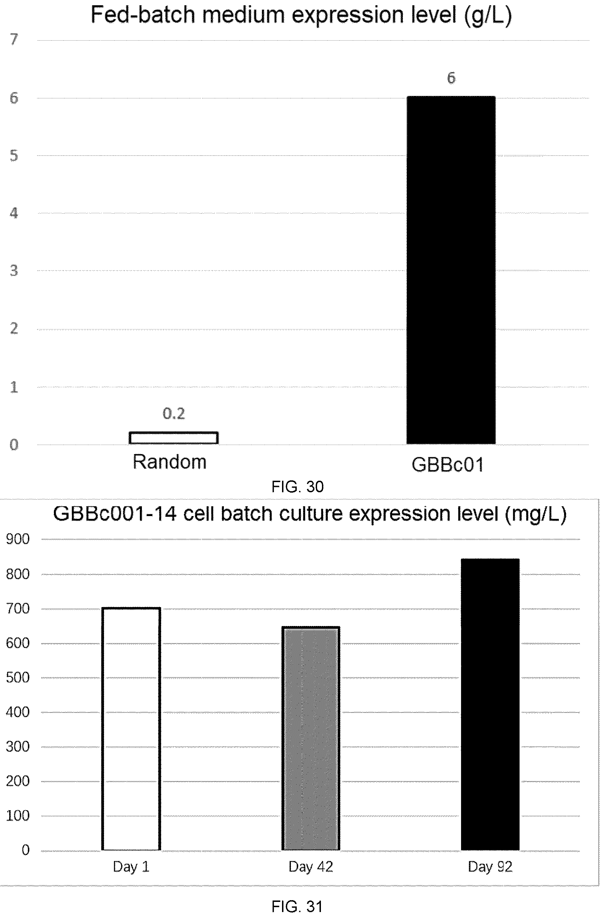

(13) In step 10, in order to investigate the subculture stability of the cell product clone corresponding to the exogenous target gene 3 expression vector microparticles, the product clone was continuously subcultured for about 90 days for stability assessment. During the continuous subculture process, the exogenous target gene 3 expression vector microparticles of the 1st, 13th and 26th generations were respectively batch cultured for 7 days (Advanced+1% GlutaMAX), and sugar was replenished on the 3rd day of the culture, the 5th day of the culture, and the 7th day of the culture according to the sugar consumption of the cells. As shown in FIG. 31, the results showed that the exogenous target gene 3 expression vector microparticles showed subculture stability and high expression level based on the expression level of target gene 3. Meanwhile, the exogenous target gene 3 expression vector microparticles were alternatively referred to as GBBc001-14 cells. The exogenous target gene 1 expression vector microparticles were alternatively referred to as GBBc001-3 cells.

The parental cells GBBc001 corresponding to GBBc001-14 cells with high expression of both fluorescent protein EGFP and target gene 3 were subjected to next-generation whole-genome sequencing, and the annotation information of the integration site on CHO-K1 was obtained as follows: NC_048595:28250698, which was located in intron 3of11 of Mtf1. In the parental cell GBBc001, the insertion position of the exogenous gene in the endogenous gene was NC_048595:28250698, and the insertion position was named as S2C4 site.

The sequence fragment after integration of the target gene 3 was as shown in SEQ ID No.19.

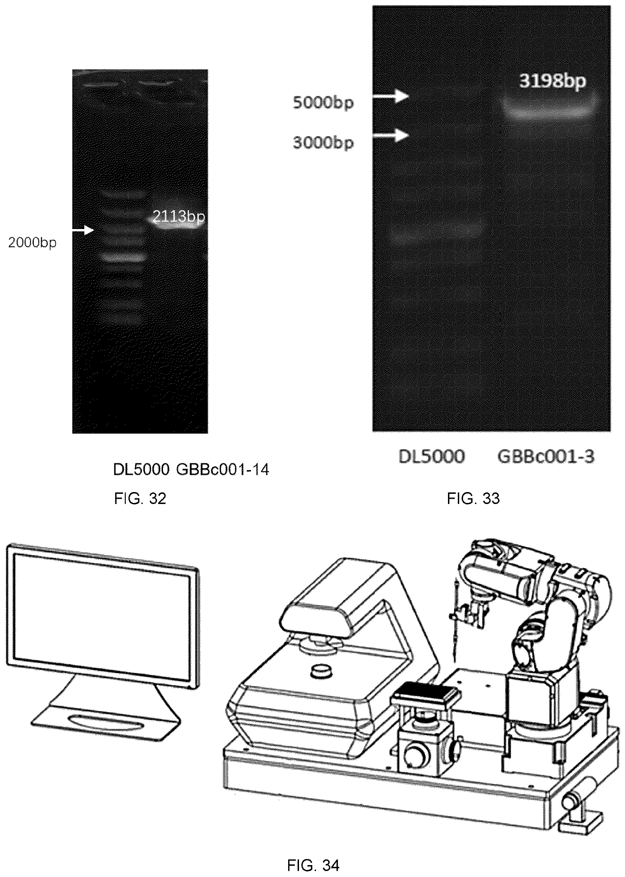

The GBBc001-14 cell genome was amplified using the breakpoint forward primer F1 (SEQ ID No. 20: gtctgg gcatggtggttgca, 131 bp upstream of the breakpoint in the genome) and the target gene 3 reverse primer R1 (SEQ ID No. 21: gaggacactccacgcaaca, approximately 600 bp downstream of the translation start site of the target gene 3) in the exogenous target gene expression fragment, to obtain a fragment with the theoretical size (2113 bp) (131 bp in the genome, 696 bp in the original vector integrated into the genome, and 1286 bp in the product sequence after site replacement), as shown in FIG. 32 (band corresponding to the GBBc001-14 lane).

The amplified band was cut and recovered from the gel, and purified, and multiple pairs of primers were designed for DNA sequencing; and after splicing the sequencing results, the sequence was as shown in SEQ ID No. 19, which was consistent with the theoretical sequence, thus confirming that the target gene 3 was correctly integrated into the corresponding position of the marker gene expression fragment.

Similarly, the sequence fragment of the cell strain GBBc001-3, which highly expressed both the fluorescent gene EGFP and the target gene 1 after being integrated with the target gene 1, was as shown in SEQ ID No. 22.

The GBBc001-3 cell genome was amplified using the breakpoint forward primer F3 (SEQ ID No. 20): gtctgg gcatggtggttgca and the target gene 1 reverse primer R3 (SEQ ID No. 23): actcgcctctattgaagct on the exogenous target gene 1 expression fragment, to obtain a fragment with the theoretical size (3198 bp), as shown in FIG. 33 (band corresponding to the GBBc001-3 lane).

The amplified band was cut and recovered from the gel, and purified, and multiple pairs of primers were designed for DNA sequencing; and after splicing the sequencing results, the sequence was as shown in SEQ ID No. 22, which was consistent with the theoretical sequence, thus confirming that the target gene 1 was correctly integrated into the corresponding position of the marker gene expression fragment.

Example 5: System for screening a site-directed integration site

For the system for screening a site-directed integration site as shown in FIG. 34, the system for screening a site-directed integration site is an electronic device and/or a non-transitory computer-readable storage medium;
the electronic device includes a display, a microscope, a robotic arm, a stage, a memory, a processor, and a computer program stored on the memory and executable on the processor, and when the processor executes the program, the steps of the method for screening a site-directed integration site as described in Examples 1 to 4 are implemented.

The non-transitory computer-readable storage medium stores a computer program thereon, and when the computer program is executed by the processor, the steps of the method for screening a site-directed integration site as described in Examples 1 to 4 are implemented.

Example 6: Site-directed integration system of an exogenous target gene

For the site-directed integration system of an exogenous target gene as shown in FIG. 33, the site-directed integration system of an exogenous target gene is an electronic device and/or a non-transitory computer-readable storage medium;
the electronic device includes a display, a microscope, a robotic arm, a stage, a memory, a processor, and a computer program stored on the memory and executable on the processor, and when the processor executes the program, the steps of the site-directed integration method of an exogenous target gene as described in Examples 1 to 4 are implemented.

The non-transitory computer-readable storage medium stores a computer program thereon, and when the computer program is executed by the processor, the steps of the site-directed integration method of the exogenous target gene as described in Examples 1 to 4 are implemented.

It will be easily understood by those skilled in the art that the above are merely preferred embodiments of the present application and not intended to limit the present application. Any modifications, equivalent substitutions, and improvements made within the spirit and principles of the present application should be included in the scope of protection of the present application.

**Claims**

1. A method for screening a site-directed integration site, comprising following steps:

   (1) randomly integrating an exogenous gene comprising two recognition sequences and a marker gene between the two recognition sequences into an endogenous gene of a vector microparticle to obtain a vector microparticle carrying the marker gene;
   (2) amplifying and culturing the vector microparticle carrying the marker gene obtained in step (1) to form a to-be-screened vector microparticle pool;
   (3) performing monoclonal screening on the to-be-screened vector microparticle pool obtained in step (2), and simultaneously during the monoclonal screening, retaining, according to an expression level of the marker gene and/or an exogenous target gene, a monoclonal vector microparticle of which the expression level of the corresponding marker gene and/or the exogenous target gene is higher than a preset threshold, to obtain the monoclonal vector microparticle with high protein expression level; and
   (4) performing subculture on the monoclonal vector microparticle with high protein expression level obtained in step (3), determining a subculture stability of the monoclonal vector microparticle according to expression levels of the marker gene in multiple generations of vector microparticles to obtain a monoclonal vector microparticle with high expression level and subculture stability, and determining an insertion position of the exogenous gene on the endogenous gene as the site-directed integration site.

2. The method for screening a site-directed integration site according to claim 1, wherein the marker gene is of a comparable length to the exogenous target gene to be integrated;

   the marker gene has multiple optional lengths, preferably 0.75k, 1k, 3k, and 15k; and
   the marker gene of 3k or less is preferably used and an expression level of an exogenous target gene of other length is predicted through an intelligent learning algorithm.

3. The method for screening a site-directed integration site according to claim 1, wherein the marker gene is selected from one or more of a neomycin resistance gene, a thymidine kinase gene, a hygromycin phosphotransferase gene, a dihydrofolate reductase gene, a thymidine kinase gene, a glutamine synthetase gene, an asparagine synthetase gene, a tryptophan synthetase gene, a histidinol dehydrogenase gene, an aminoglycoside phosphotransferase gene, a tryptophan synthetase gene, and a fluorescent protein gene; and/or,

   the vector microparticle is configured to express one or more of an amino acid, a polypeptide, a protein, a nucleotide, a nucleic acid, and a secretion, is one selected from a cell, a bacterium, and a virus, and is commonly a vector cell; the vector cell is an eukaryotic cell; optionally, the eukaryotic cell is a mammalian cell; optionally, the mammalian cell comprises a Chinese hamster ovary CHO cell and a human embryonic kidney HEK293 cell; and/or,
   the recognition sequences are each independently selected from one or more of following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a Bxb1 attP sequence, a Bxb1 attB sequence, an attP sequence, an attB sequence, an attP-GA sequence and an attB-GA sequence.

4. The method for screening a site-directed integration site according to claim 1, wherein in step (3), the monoclonal screening of the vector microparticle is performed using an image processing technology, a specific solution of which comprises but not limited to deep learning object detection models which are algorithm models of YOLO series and SSD series.

5. The method for screening a site-directed integration site according to claim 4, wherein the expression level of the marker gene and/or the exogenous target gene in step (3) is predicted by an intelligent algorithm according to a cell image, specifically comprising following steps:

(3-1) imaging the to-be-screened vector microparticle pool obtained in step (2) to acquire to-be-screened vector microparticle images, wherein preferably high-throughput scanning imaging is used, and preferably high-throughput fluorescence scanning imaging is used when the marker gene is a fluorescent protein;

(3-2) performing image processing on the to-be-screened vector microparticle images obtained in step (3-1) to obtain an image of each vector microparticle, and predicting the expression level of the marker gene and/or the exogenous target gene of the vector microparticle by using the intelligent algorithm according to image features, wherein preferably, the image features used comprise: cell morphological features and/or optical features, comprising but not limited to roundness, size, and grayscale of a specific cell site;

preferably, for the expression levels of the marker gene and/or the exogenous target gene of the vector microparticle predicted by multiple intelligent algorithms, normalized discounted cumulative gain, an evaluation standard of a ranking algorithm, is used to evaluate ranking accuracy of protein expression levels predicted by the multiple intelligent algorithms, according to a rule that the larger a standard normalized discounted cumulative gain, the better the ranking accuracy of the protein expression level, and an intelligent algorithm with best ranking accuracy in predicted protein expression levels is selected;

for a specific intelligent algorithm for predicting the protein expression level, a calculation formula of the standard normalized discounted cumulative gain is as follows:

$$DCG = \sum_{i=1}^{k} \frac{rel(i)}{\log_2(i+1)}$$

$$NDCG = \frac{DCG}{IDCG}$$

wherein IDCG indicates a DCG value of an actual ranking of protein expression levels of sample monoclonal vector microparticles, i indicates a predicted ranking number of a monoclonal vector microparticle in the sample monoclonal vector microparticles obtained according to ranking of the protein expression levels predicted by the intelligent algorithm, rel(i) indicates an actual protein expression level of an i-th monoclonal vector microparticle; and k indicates a sample size of the sample monoclonal vector microparticles;

wherein when a predicted protein is a fluorescent protein, rel(i) indicates a fluorescence value; when the predicted protein is a non-fluorescent protein, rel(i) indicates a value of a protein expression level tested by Western blot, ELISA, mass spectrometry, electrophoresis, etc.; and

(3-3) acquiring, according to image localization, the vector microparticle of which the expression level of the marker gene and/or the exogenous target gene exceeds the preset threshold, and moving to a preset position in a medium for retention.

6. The method for screening a site-directed integration site according to claim 1, wherein in step (3), a semi-solid medium monoclonal vector microparticle screening is used.

7. The method for screening a site-directed integration site according to claim 1, wherein in step (4), the subculture stability of the monoclonal vector microparticle is determined by using an intelligent algorithm, comprising following specific steps:

(4-1) acquiring expression levels of the marker gene of multiple generations of monoclonal vector microparticles;
(4-2) evaluating differences in the expression levels of the marker gene of the multiple generations of monoclonal vector microparticles by using the intelligent algorithm according to the expression levels of the marker gene of the multiple generations of monoclonal vector microparticles obtained in step (4-1); and
(4-3) determining whether the monoclonal vector microparticle has subculture stability, based on the differences in the expression levels of the marker gene of the multiple generations of monoclonal vector microparticles obtained in step (4-2), according to a principle that the smaller a difference in the expression level, the greater a probability of having subculture stability.

8. A site-directed integration vector microparticle obtained by screening through the method for screening a site-directed integration site according to any one of claims 1 to 7, wherein the exogenous gene is inserted into the endogenous gene of the vector microparticle; the exogenous gene comprises two first recognition sequences and a marker gene expression fragment configured for expressing a marker protein and integrated between the two first recognition sequences;

an expression level of the marker gene of the site-directed integration vector microparticle is higher than the preset threshold; and
the site-directed integration vector microparticle has subculture stability.

9.  A system for screening a site-directed integration site, wherein the system is an electronic device and/or a non-transitory computer-readable storage medium;

the electronic device comprises a memory, a processor, and a computer program stored on the memory and executable on the processor, wherein when the processor executes the program, steps of the method for screening a site-directed integration site according to any one of claims 1 to 7 are implemented; and
the non-transitory computer-readable storage medium stores a computer program thereon, wherein when the computer program is executed by the processor, the steps of the method for screening a site-directed integration site according to any one of claims 1 to 7 are implemented.

10. A site-directed integration method of an exogenous target gene, comprising following steps:
acquiring the site-directed integration vector microparticle according to claim 8, and performing homologous recombination by using the first recognition sequences in the exogenous gene in the site-directed integration vector microparticle, to recombine at least one exogenous target gene expression fragment into a gene of the site-directed integration vector microparticle and simultaneously remove a marker gene expression fragment.

11. The site-directed integration method of an exogenous target gene according to claim 10, wherein the exogenous target gene expression fragment is provided in a recombinant vector, and the at least one exogenous target gene is site-specifically integrated into the site of the site-directed integration vector microparticle through the recombinant vector; the recombinant vector is a lentiviral vector, an adenoviral vector, an adeno-associated viral vector, a herpesvirus vector, a poxvirus vector, a baculovirus vector, a papillomavirus vector, a papovavirus vector, an integrating phage vector, a non-viral vector, a transposon, a transposase, an integrase substrate or a plasmid.

12. The site-directed integration method of an exogenous target gene according to claim 10, wherein a length of the marker gene expression fragment of the site-directed integration vector microparticle is comparable to a length of the at least one exogenous target gene expression fragment.

13. The site-directed integration method of an exogenous target gene according to claim 10, wherein the performing homologous recombination by using the first recognition sequences of the site-directed integration fragment of the site-directed integration vector microparticle specifically comprises:

constructing an exogenous target gene recombinant fragment comprising an exogenous target gene, wherein the at least one exogenous gene recombinant fragment comprises two second recognition sequences and an exogenous target gene expression fragment; the at least one exogenous target gene expression fragment is located between the two second recognition sequences; and the at least one exogenous target gene expression fragment includes a promoter configured for regulating expression of the exogenous target gene and an exogenous target gene sequence; and
integrating the exogenous gene into the site of the site-directed integration vector microparticle, wherein the two first recognition sequences on two sides of a marker gene are respectively changed into a third recognition sequence and a fourth recognition sequence; the third recognition sequence is formed by reaction of the first recognition sequence and the second recognition sequence, and the fourth recognition sequence is formed by reaction of the first recognition sequence and the second recognition sequence, and the third recognition sequence is different from the fourth recognition sequence.

14. The site-directed integration method of an exogenous target gene according to claim 13, wherein the third recognition sequence and the fourth recognition sequence both are not changed into the second recognition sequence or the first recognition sequence in a same reaction system.

15. The site-directed integration method of an exogenous target gene according to claim 13, wherein the third recognition sequence and the fourth recognition sequence are generated by a recombinase, and the recombinase is one of a Bxb1 integrase, a ΦC31 integrase, Cre recombinase, or a FLP recombinase; and/or

the second recognition sequence is independently selected from one or more of following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a Bxb1 attP sequence, a Bxb1 attB sequence, an attP sequence, an attB sequence, an attP-GA sequence and an attB-GA sequence; and/or

wherein the third recognition sequence and the recognition sequence are each independently selected from one or more of following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, an attL sequence and an attR sequence.

16. The site-directed integration method of an exogenous target gene according to claim 10, comprising following steps:

S1. acquiring a plurality of site-directed integration vector microparticles according to claim 8, and performing homologous recombination by using the first recognition sequences in the exogenous genes in the site-directed integration vector microparticles, to recombine the exogenous target gene expression fragments into the genes of the site-directed integration vector microparticles and simultaneously remove the marker gene expression fragments to obtain multiple candidate exogenous target gene expression vector microparticles; and

S2. amplifying and subculturing the multiple candidate exogenous target gene expression vectors obtained in step S1;

S21. retaining, according to an expression level of the exogenous target gene, monoclonal exogenous target gene expression vector microparticles of which the expression level of the corresponding exogenous target gene is higher than a preset threshold;

S22. determining subculture stabilities of the exogenous target gene expression vector microparticles according to expression levels of the exogenous target gene of multiple generations of monoclonal exogenous target gene expression vector microparticles; and

obtaining exogenous target gene expression vector microparticles with high protein expression level and subculture stability.

17. The site-directed integration method of an exogenous target gene according to claim 16, wherein in step S21, the expression level of the exogenous target gene is predicted by an intelligent learning algorithm; and

expression level retention of the exogenous target gene is predicted by an intelligent algorithm according to a cell image, specifically comprising following steps:

S211. imaging the monoclonal exogenous target gene expression vector microparticles obtained in step S21 to acquire images of candidate monoclonal exogenous target gene expression vector microparticles, wherein preferably, high-throughput scanning imaging is used; and

S212. predicting the expression levels of the exogenous target gene in the monoclonal exogenous target gene expression vector microparticles using the intelligent algorithm according to image features, wherein preferably, the image features used comprise: cell morphological features and/or optical features, comprising but not limited to roundness, size, and grayscale of a specific cell site,

wherein preferably, for the expression levels of the exogenous target gene of the monoclonal exogenous target gene expression vector microparticles predicted by multiple intelligent algorithms, normalized discounted cumulative gain, an evaluation standard of the ranking algorithm, is used to evaluate a ranking accuracy of protein expression levels predicted by the multiple intelligent algorithms, according to a rule that the greater a standard normalized discounted cumulative gain, the better the ranking accuracy of the protein expression levels, and an intelligent algorithm with best ranking accuracy in predicted protein expression levels is selected;

for a specific intelligent algorithm for predicting the protein expression level, a calculation formula of the standard normalized discounted cumulative gain is as follows:

$$DCG = \sum_{i=1}^{k} \frac{rel(i)}{\log_2(i+1)}$$

$$NDCG = \frac{DCG}{IDCG}$$

wherein IDCG indicates a DCG value of an actual ranking of protein expression levels of sample monoclonal vector microparticles, i indicates a predicted ranking number of a monoclonal vector microparticle in the sample monoclonal vector microparticles obtained according to ranking of protein expression levels predicted by the intelligent algorithm, rel(i) indicates an actual protein expression level of an i-th monoclonal vector microparticle; and k indicates a sample size of the sample monoclonal vector microparticles; and

when a predicted protein is a fluorescent protein, rel(i) indicates a fluorescence value; and when the predicted protein is a non-fluorescent protein, rel (i) indicates a value of the protein expression level tested by Western blot, ELISA, mass spectrometry analysis, electrophoresis, etc.

18. The site-directed integration method of an exogenous target gene according to claim 16, wherein in step S22, subculture stabilities of the monoclonal exogenous target gene expression vector microparticles are determined by using an intelligent algorithm, comprising specific steps as follows:

S221. acquiring the expression levels of the exogenous target gene of multiple generations of the monoclonal exogenous target gene expression vector microparticles;

S222. evaluating the differences in the expression levels of the exogenous target gene in the multiple generations of monoclonal exogenous target gene expression vector microparticles by using the intelligent algorithm according to the expression levels of the exogenous target gene of the multiple generations of monoclonal exogenous target gene expression vector microparticles obtained in step S221; and

S223. determining whether the monoclonal exogenous target gene expression vector microparticles have subculture stabilities, based on the differences in the expression levels of the exogenous target gene of the multiple generations of monoclonal exogenous target gene expression vector microparticles obtained in step S222, according to a principle that the smaller a difference in the expression levels, the greater a probability of having the subculture stability.

19. An exogenous target gene expression vector microparticle obtained by screening through the site-directed integration method of an exogenous target gene according to any one of claims 10 to 18, wherein the exogenous target gene is inserted into an endogenous gene of the vector microparticle; the exogenous gene comprises a third recognition sequence, a fourth recognition sequence, and at least one exogenous target gene expression fragment configured for a target protein and arranged between the third recognition sequence and the fourth recognition sequence;

a target gene expression level of the exogenous target gene expression vector microparticle is higher than the preset threshold; and

the exogenous target gene expression vector microparticle has subculture stability.

20. A site-directed integration system of an exogenous target gene, wherein the system is an electronic device and/or a non-transitory computer-readable storage medium;

the electronic device comprises a memory, a processor, and a computer program stored on the memory and executable on the processor, wherein when the processor executes the program, steps of the site-directed integration method of an exogenous target gene according to any one of claims 10 to 18 are implemented; and the non-transitory computer-readable storage medium stores the computer program thereon, and when the computer program is executed by the processor, the steps of the site-directed integration method of an exogenous target gene according to any one of claims 10 to 18 are implemented.

FIG. 1

FIG. 2

Start

Transferring cells to a culture dish containing semi-solid medium, and standing to waiting for the cells to settle to the bottom of the culture dish

Electron microscope performing high-throughput scanning on the culture dish

Predicting and ranking the protein expression levels of monoclonal cell strains by artificial intelligence to obtain information on high-expression cell strains

Returning the information on screened high-expression cell strains to robot control software

Transferring all screened monoclonal cell strains to the designated well plates by the robot

End

Using the screened stable high-expression cell strains as the final candidate cell strains

Performing expression level stability prediction on the photographed cell strain images and screening out stable high-expression cell strains

Subculturing the high-expression monoclonal cell strains and photographing cell strain images

Expanding and culturing the selected high-expression monoclonal cell strains, and checking the expression levels to determine high yield

FIG. 3

FIG. 4

Introducing the selection marker expression RMCE
cassette into a CHO host by random integration

↓ clonal selection
and expansion

Screening for host monoclone with high
selection marker and low copy number

↓

Stability assessment of marker gene cell

↓

Product assessment for stable cells with
high selection marker

↓

The product gene undergoing sequence exchange with the selection marker
expression RMCE cassette in the host through homologous recombination

↓

Proving that the host integration site is a high-expression
and stable site for the product

↓

The candidate host cell strains being subjected to methods
such as sequencing to obtain integration site information

FIG. 5

FIG. 6

FIG. 7

D14 expression level (mg/L)

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

## Glycosylation profile peak comparison

FIG. 26

## Charge variation comparison

FIG. 27

5000bp →
3000bp →
2000bp →

2876bp

DL5000       GBB-01-H8

FIG. 28

Fed-batch medium expression level (g/L)

FIG. 29

FIG. 30

FIG. 31

DL5000 GBBc001-14

FIG. 32

DL5000    GBBc001-3

FIG. 33

FIG. 34

## INTERNATIONAL SEARCH REPORT

| International application No. |
| --- |
| **PCT/CN2024/092536** |

**A. CLASSIFICATION OF SUBJECT MATTER**

C12N15/65(2006.01)i; C12N15/66(2006.01)i; C12N15/85(2006.01)i; C12N5/10(2006.01)i

According to International Patent Classification (IPC) or to both national classification and IPC

**B. FIELDS SEARCHED**

Minimum documentation searched (classification system followed by classification symbols)

IPC:C12N

Documentation searched other than minimum documentation to the extent that such documents are included in the fields searched

Electronic data base consulted during the international search (name of data base and, where practicable, search terms used)

CNTXT, ENTXTC, VCN, VEN, PUBMED, NCBI, CNKI, BAIDU, BING: 定点整合, 整合位点, 筛选, 标记基因, 表达量, 识别序列, 同源重组, LoxP, Lox, attB, attP, targeted integration, site-specific, site-directed, integration site, marker gene, screen, selection, expression, homologous recombination

**C. DOCUMENTS CONSIDERED TO BE RELEVANT**

| Category* | Citation of document, with indication, where appropriate, of the relevant passages | Relevant to claim No. |
| --- | --- | --- |
| X | CN 106978416 A (SHANGHAI GENON BIOENGINEERING CO., LTD. et al.) 25 July 2017 (2017-07-25) abstract and claims 1-10 | 8,19 |
| A | CN 1693467 A (BEIJING TIANGUANGSHI BIO-TECHNOLOGY CO., LTD.) 09 November 2005 (2005-11-09) claims 1-35 | 1-20 |
| A | CN 104673815 A (SOUTHWEST UNIVERSITY) 03 June 2015 (2015-06-03) entire document | 1-20 |
| A | US 2021002669 A1 (GENENTECH, INC.) 07 January 2021 (2021-01-07) entire document | 1-20 |
| A | US 2012124686 A1 (LUO, L. Q. et al.) 17 May 2012 (2012-05-17) entire document | 1-20 |
| A | US 2023159958 A1 (CYTIVA SWEDEN AB) 25 May 2023 (2023-05-25) entire document | 1-20 |

☐ Further documents are listed in the continuation of Box C.　　☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **31 July 2024** | **06 August 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/092536** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

    ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

## INTERNATIONAL SEARCH REPORT
### Information on patent family members

International application No.

**PCT/CN2024/092536**

| Patent document cited in search report | | | Publication date (day/month/year) | Patent family member(s) | | | Publication date (day/month/year) |
|---|---|---|---|---|---|---|---|
| CN | 106978416 | A | 25 July 2017 | None | | | |
| CN | 1693467 | A | 09 November 2005 | None | | | |
| CN | 104673815 | A | 03 June 2015 | None | | | |
| US | 2021002669 | A1 | 07 January 2021 | WO | 2019126634 | A2 | 27 June 2019 |
| | | | | WO | 2019126634 | A3 | 15 August 2019 |
| | | | | JP | 2021507697 | A | 25 February 2021 |
| | | | | BR | 112020012591 | A2 | 24 November 2020 |
| | | | | TW | 201932600 | A | 16 August 2019 |
| | | | | EP | 3728309 | A2 | 28 October 2020 |
| | | | | KR | 20200103765 | A | 02 September 2020 |
| | | | | MX | 2020006117 | A | 10 February 2022 |
| | | | | JP | 2022070949 | A | 13 May 2022 |
| | | | | AU | 2018392728 | A1 | 09 July 2020 |
| | | | | CA | 3083579 | A1 | 27 June 2019 |
| | | | | IL | 275462 | A | 31 August 2020 |
| | | | | SG | 11202005109 | SA | 29 July 2020 |
| US | 2012124686 | A1 | 17 May 2012 | WO | 2012064970 | A1 | 18 May 2012 |
| | | | | US | 9125385 | B2 | 08 September 2015 |
| US | 2023159958 | A1 | 25 May 2023 | JP | 2023520947 | A | 22 May 2023 |
| | | | | WO | 2021204807 | A1 | 14 October 2021 |
| | | | | AU | 2021252114 | A1 | 03 November 2022 |
| | | | | EP | 4133089 | A1 | 15 February 2023 |
| | | | | GB | 202005180 | D0 | 20 May 2020 |
| | | | | KR | 20220164765 | A | 13 December 2022 |

Form PCT/ISA/210 (patent family annex) (July 2022)

**REFERENCES CITED IN THE DESCRIPTION**

**Patent documents cited in the description**

- CN 100381573 C
- CN 114107380 A
- CN 113821287 B
- CN 113771030 A
- CN 113733087 B